# EUROPEAN PATENT APPLICATION

(11) **EP 4 729 067 A2**
(43) Date of publication of application: **22.04.2026**
(21) Application number: 26163081.8
(22) Date of filing: 04.08.2022
(51) Int. Cl.: A61K 39/00

(54) **ELECTRODE ASSEMBLY FOR APPLYING TUMOR TREATING FIELDS WITH A SHEET OF ANISOTROPIC MATERIAL**

(30) Priority: 06.08.2021 US 202163230438 P; 04.11.2021 US 202163275841 P; 04.11.2021 US 202163275843 P
(62) Divisional of application: 22757665.9
(71) Applicant: Novocure GmbH, 6340 Baar (CH)
(72) Inventor: WASSERMAN, Yoram, 3190500 Haifa (IL); OBUCHOVSKY, Stas, 3190500 Haifa (IL); KUPLENNIK, Nataliya, 3190500 Haifa (IL); SHAPIRO, David, 3190500 Haifa (IL)
(74) Representative: Boden, Keith McMurray

(57) **Abstract**

An apparatus for applying an alternating electric field to a subject's body, the apparatus comprising: a sheet of anisotropic material (70) having a front face and a rear face, the front face being configured to face the subject's skin, wherein the sheet of anisotropic material (70) has a first resistance in a direction that is perpendicular to the front face thereof, and a resistance of the sheet of anisotropic material (70) in directions that are parallel to the front face thereof is less than half of the first resistance; at least one layer of conductive material (60) disposed in electrical contact with the front face of the sheet of anisotropic material (70), wherein the at least one layer of conductive material (60) has a biocompatible front surface; and at least one electrode element (E1, E2) positioned behind the sheet of anisotropic material (70), wherein the at least one electrode element (E1, E2) has a front face disposed in electrical contact with the rear face of the sheet of anisotropic material (70).

## Description

### CROSS REFERENCE TO RELATED APPLICATIONS

This Application claims the benefit of US Provisional Applications 63/230,438 (filed August 6, 2021), 63/275,841 (filed November 4, 2021), and 63/275,843 (filed November 4, 2021), each of which is incorporated herein by reference in its entirety.

### BACKGROUND

Tumor Treating Fields (TTFields) therapy is a proven approach for treating tumors using alternating electric fields at frequencies between 50 kHz and 1 MHz, such as, for example, 100-500 kHz. The alternating electric fields are induced by electrode assemblies (e.g., arrays of capacitively coupled electrodes, also called transducer arrays) placed on opposite sides of the subject's body. When an AC voltage is applied between opposing electrode assemblies, an AC current is coupled through the electrode assemblies and into the subject's body. And higher currents are strongly correlated with higher efficacy of treatment.

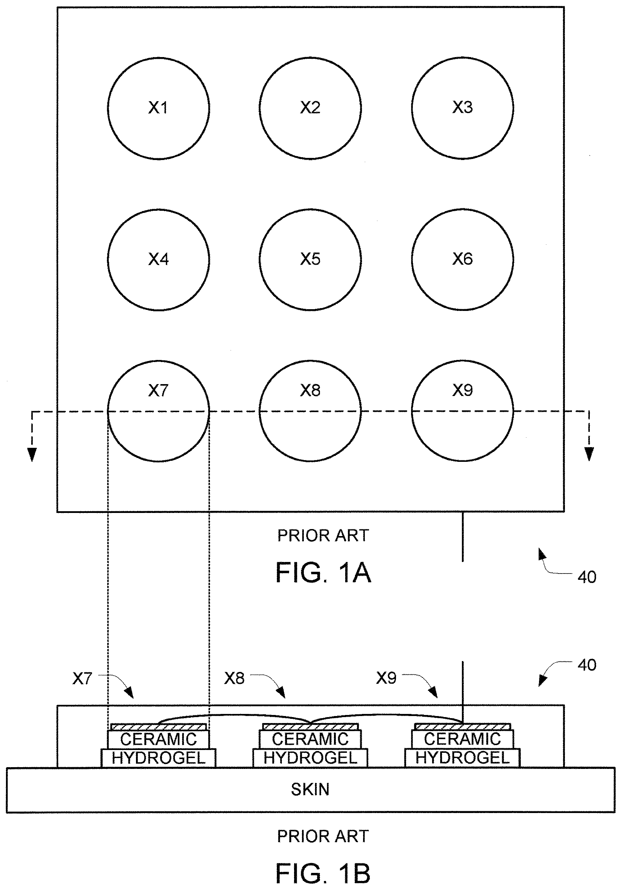

FIG. 1A is a schematic representation of a prior art electrode assembly 40 including nine prior art electrode elements, labeled X1-X9. FIG. 1B is a cross sectional schematic view of electrode elements X7-X9 of the electrode assembly 40, taken along the dashed line in FIG. 1A.

As shown in FIG. 1B, electrode element X7 (taken as exemplary) includes a metal layer (shown with diagonal hatching) and a ceramic (dielectric) layer. A respective layer of electrically conductive hydrogel is provided between each ceramic layer and the subject's skin, to ensure good electrical contact of the electrode elements with the body. An AC voltage from an AC voltage generator (not shown) is applied to the metal layers of electrode elements in opposing electrode assemblies to generate the TTFields in the subject's body.

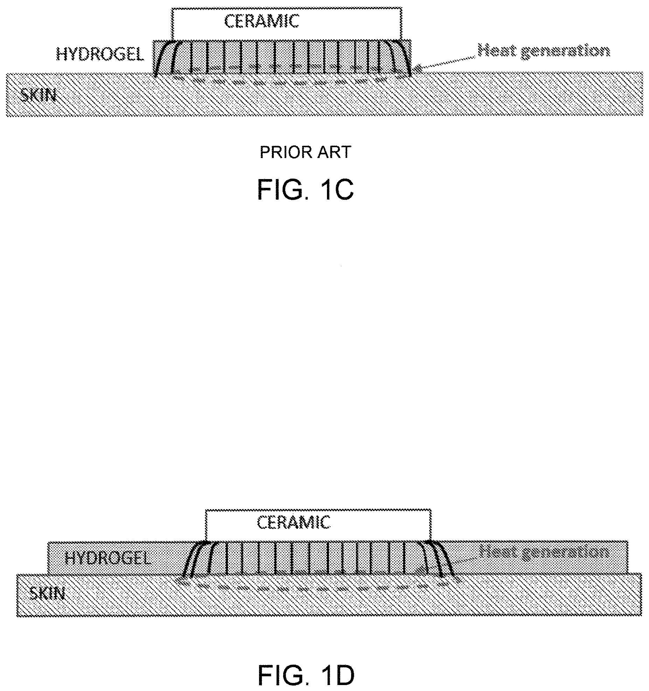

During use, the hydrogel and the skin under the electrode elements heat up, and safety considerations require that the skin temperature remain below a safety threshold (e.g., 41° C). Because the vast majority of the heat appears immediately below the electrode elements X1-X9 (as shown in FIG. 1C), the prior art electrode assembly has hot spots immediately below the electrode elements, and cooler regions positioned between the electrode elements. And those hot spots limit the amount of current that can be delivered through the prior art electrode assemblies.

### SUMMARY OF THE INVENTION

One aspect of the invention is directed to a first apparatus for applying an alternating electric field to a subject's body. The first apparatus comprises a sheet of anisotropic material, at least one layer of conductive material, and a first electrode element. The sheet of anisotropic material has a front face and a rear face. The sheet has a first thermal conductivity in a direction that is perpendicular to the front face, and thermal conductivity of the sheet in directions that are parallel to the front face is more than two times higher than the first thermal conductivity. The at least one layer of conductive material is disposed on the front face of the sheet and has a biocompatible front surface. The first electrode element is positioned behind the sheet, and the first electrode element has a first front face disposed in electrical contact with the rear face of the sheet.

In some embodiments of the first apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, wherein the first rear layer of conductive material facilitates the electrical contact between the first front face of the first electrode element and the rear face of the sheet. Optionally, in these embodiments the first rear layer of conductive material may comprise conductive hydrogel.

In some embodiments of the first apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, wherein the first rear layer of conductive material facilitates the electrical contact between the first front face of the first electrode element and the rear face of the sheet. These embodiments also further comprise a second electrode element positioned behind the sheet. The second electrode element has a second front face disposed in electrical contact with the rear face of the sheet. The second electrode element comprises (i) a second layer of dielectric material having a front face and a rear face and (ii) a second layer of metal disposed on the rear face of the second layer of dielectric material. The front face of the second layer of dielectric material is the second front face of the second electrode element. The first rear layer of conductive material is positioned between the second front face of the second electrode element and the rear face of the sheet, and the first rear layer of conductive material facilitates the electrical contact between the second front face of the second electrode element and the rear face of the sheet.

In some embodiments of the first apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, wherein the first rear layer of conductive material facilitates the electrical contact between the first front face of the first electrode element and the rear face of the sheet. These embodiments also further comprise a second electrode element positioned behind the sheet. The second electrode element has a second front face disposed in electrical contact with the rear face of the sheet. The second electrode element comprises (i) a second layer of dielectric material having a front face and a rear face and (ii) a second layer of metal disposed on the rear face of the second layer of dielectric material. The front face of the second layer of dielectric material is the second front face of the second electrode element. These embodiments also further comprise a second rear layer of conductive material positioned between the second front face of the second electrode element and the rear face of the sheet. The second rear layer of conductive material facilitates the electrical contact between the second front face of the second electrode element and the rear face of the sheet.

In some embodiments of the first apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, wherein the first rear layer of conductive material facilitates the electrical contact between the first front face of the first electrode element and the rear face of the sheet. In these embodiments, the first rear layer of conductive material is a conductive adhesive. Optionally, in these embodiments, the conductive adhesive may comprise an adhesive polymer and carbon powder, particles, fibers, flakes or nanotubes. Optionally, in these embodiments, the conductive adhesive has a thickness between 10 and 2,000 µm.

In some embodiments of the first apparatus, the first electrode element comprises a piece of metal having a front face, and the front face of the piece of metal is the first front face of the first electrode element. Optionally, these embodiments may further comprise a first rear layer of conductive material positioned between the first front face of the first electrode element and the rear face of the sheet, wherein the first rear layer of conductive material facilitates the electrical contact between the first front face of the first electrode element and the rear face of the sheet. Optionally, in these embodiments, the first front face of the first electrode element may be positioned in direct contact with the rear face of the sheet.

In some embodiments of the first apparatus, the sheet of anisotropic material is a sheet of graphite. In some embodiments of the first apparatus, the sheet of anisotropic material is a sheet of pyrolytic graphite, graphite foil made from compressed high purity exfoliated mineral graphite, or graphitized polymer film. In some embodiments of the first apparatus, the sheet of anisotropic material is nonmetallic.

In some embodiments of the first apparatus, the thermal conductivity of the sheet in directions that are parallel to the front face is more than ten times higher than the first thermal conductivity. In some embodiments of the first apparatus, the sheet has a first resistance in a direction that is perpendicular to the front face, and resistance of the sheet in directions that are parallel to the front face is less than half of the first resistance.

In some embodiments of the first apparatus, the at least one layer of conductive material comprises hydrogel. In some embodiments of the first apparatus, the at least one layer of conductive material comprises a layer of hydrogel with a thickness between 50 and 2000 µm.

In some embodiments of the first apparatus, the at least one layer of conductive material comprises a conductive adhesive. Optionally, in these embodiments, the conductive adhesive may comprise an adhesive polymer and carbon powder, particles, fibers, flakes or nanotubes. Optionally, in these embodiments, the conductive adhesive has a thickness between 10 and 2,000 µm.

Some embodiments of the first apparatus further comprise a flexible self-adhesive backing configured to support the sheet, the first electrode element, and the at least one layer of conductive material so that the front surface of the at least one layer of conductive material can be positioned against a subject's skin.

In some embodiments of the first apparatus, the sheet has a centroid, and a centroid of the first front face of the first electrode element is positioned less than 3 cm away from the centroid of the sheet. In some embodiments of the first apparatus, the sheet has a centroid and a dimension in a direction parallel to the rear face of the sheet, and a centroid of the first front face of the first electrode element is positioned away from the centroid of the sheet by less than 30% of the dimension.

Some embodiments of the first apparatus further comprise a lead that is electrically connected to the first electrode element.

Another aspect of the invention is directed to a first method of applying an alternating electric field to a target region in a subject's body. The first method comprises positioning a first electrode assembly at a first position on or in the subject's body. The first electrode assembly includes a first sheet of anisotropic material having a first front face and a first rear face. The first sheet has a first thermal conductivity in a direction that is perpendicular to the first front face. Thermal conductivity of the first sheet in directions that are parallel to the first front face is more than two times higher than the first thermal conductivity. The first electrode assembly is positioned so that the first front face of the first sheet faces the target region. The first method also comprises positioning a second electrode assembly at a second position on or in the subject's body. The second electrode assembly includes a second sheet of anisotropic material having a second front face and a second rear face. The second sheet has a second thermal conductivity in a direction that is perpendicular to the second front face. Thermal conductivity of the second sheet in directions that are parallel to the second front face is more than two times higher than the second thermal conductivity. The second electrode assembly is positioned so that the second front face of the second sheet faces the target region. The first method also comprises applying an alternating voltage between the first electrode assembly and the second electrode assembly. The applying is performed after positioning the first electrode assembly and the second electrode assembly.

In some instances of the first method, the applying is implemented by applying the alternating voltage between (i) a first electrode element disposed in electrical contact with the first rear face and (ii) a second electrode element disposed in electrical contact with the second rear face.

Some instances of the first method further comprise measuring a first temperature of the first electrode element; measuring a second temperature of the second electrode element; and controlling the applying based on the first temperature and the second temperature.

In some instances of the first method, the first electrode assembly further includes a first layer of conductive material disposed on the first front face. In these instances, the second electrode assembly further includes a second layer of conductive material disposed on the second front face.

In some instances of the first method, each of the first and second sheets of anisotropic material is a sheet of graphite. In some instances of the first method, each of the first and second sheets of anisotropic material is a sheet of pyrolytic graphite, graphite foil made from compressed high purity exfoliated mineral graphite, or graphitized polymer film. In some instances of the first method, each of the first and second sheets of anisotropic material is nonmetallic.

In some instances of the first method, the thermal conductivity of the first sheet in directions that are parallel to the first front face is more than ten times higher than the first thermal conductivity, and thermal conductivity of the second sheet in directions that are parallel to the second front face is more than ten times higher than the second thermal conductivity.

In some instances of the first method, the first sheet has a first resistance in a direction that is perpendicular to the first front face, and resistance of the first sheet in directions that are parallel to the first front face is less than half of the first resistance. The second sheet has a second resistance in a direction that is perpendicular to the second front face, and resistance of the second sheet in directions that are parallel to the second front face is less than half of the second resistance.

Another aspect of the invention is directed to a second apparatus for applying an alternating electric field to a subject's body. The second apparatus comprises a sheet of anisotropic material, at least one layer of conductive material, and a first electrode element. The sheet of anisotropic material has a front face and a rear face. The sheet has a first resistance in a direction that is perpendicular to the front face, and resistance of the sheet in directions that are parallel to the front face is less than half of the first resistance. The at least one layer of conductive material is disposed on the front face of the sheet and has a biocompatible front surface. The first electrode element is positioned behind the sheet, and the first electrode element has a first front face disposed in electrical contact with the rear face of the sheet.

In some embodiments of the second apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, and the first rear layer of conductive material facilitates the electrical contact between the first front face of the first electrode element and the rear face of the sheet. Optionally, in these embodiments, the first rear layer of conductive material may comprise conductive hydrogel.

In some embodiments of the second apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, and the first rear layer of conductive material facilitates the electrical contact between the first front face of the first electrode element and the rear face of the sheet. These embodiments also further comprise a second electrode element positioned behind the sheet. The second electrode element has a second front face disposed in electrical contact with the rear face of the sheet. The second electrode element comprises (i) a second layer of dielectric material having a front face and a rear face and (ii) a second layer of metal disposed on the rear face of the second layer of dielectric material. The front face of the second layer of dielectric material is the second front face of the second electrode element. The first rear layer of conductive material is positioned between the second front face of the second electrode element and the rear face of the sheet, and the first rear layer of conductive material facilitates the electrical contact between the second front face of the second electrode element and the rear face of the sheet.

In some embodiments of the second apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, and the first rear layer of conductive material facilitates the electrical contact between the first front face of the first electrode element and the rear face of the sheet. These embodiments also further comprise a second electrode element positioned behind the sheet. The second electrode element has a second front face disposed in electrical contact with the rear face of the sheet. The second electrode element comprises (i) a second layer of dielectric material having a front face and a rear face and (ii) a second layer of metal disposed on the rear face of the second layer of dielectric material. The front face of the second layer of dielectric material is the second front face of the second electrode element. The apparatus further comprises a second rear layer of conductive material positioned between the second front face of the second electrode element and the rear face of the sheet, and the second rear layer of conductive material facilitates the electrical contact between the second front face of the second electrode element and the rear face of the sheet.

In some embodiments of the second apparatus, the first electrode element comprises (i) a first layer of dielectric material having a front face and a rear face and (ii) a first layer of metal disposed on the rear face of the first layer of dielectric material. The front face of the first layer of dielectric material is the first front face of the first electrode element. These embodiments further comprise a first rear layer of conductive material positioned between the first front face of the first electrode element and the rear face of the sheet, and the first rear layer of conductive material facilitates the electrical contact between the first front face of the first electrode element and the rear face of the sheet. In these embodiments, the first rear layer of conductive material comprises a conductive adhesive. Optionally, in these embodiments, the conductive adhesive may comprise an adhesive polymer and carbon powder, particles, fibers, flakes or nanotubes. Optionally, in these embodiments, the conductive adhesive has a thickness between 10 and 2,000 µm.

In some embodiments of the second apparatus, the first electrode element comprises a piece of metal having a front face, and the front face of the piece of metal is the first front face of the first electrode element. Optionally, these embodiments may further comprise a first rear layer of conductive material positioned between the first front face of the first electrode element and the rear face of the sheet, wherein the first rear layer of conductive material facilitates the electrical contact between the first front face of the first electrode element and the rear face of the sheet. Optionally, in these embodiments, the first front face of the first electrode element may be positioned in direct contact with the rear face of the sheet.

In some embodiments of the second apparatus, the sheet of anisotropic material is a sheet of graphite. In some embodiments of the second apparatus, the sheet of anisotropic material is a sheet of pyrolytic graphite, graphite foil made from compressed high purity exfoliated mineral graphite, or graphitized polymer film. In some embodiments of the second apparatus, the sheet of anisotropic material is nonmetallic.

In some embodiments of the second apparatus, the resistance of the sheet in directions that are parallel to the front face is less than 10% of the first resistance.

In some embodiments of the second apparatus, the sheet has a first thermal conductivity in a direction that is perpendicular to the front face, and thermal conductivity of the sheet in directions that are parallel to the front face is more than two times higher than the first thermal conductivity.

In some embodiments of the second apparatus, the at least one layer of conductive material comprises hydrogel. In some embodiments of the second apparatus, the at least one layer of conductive material comprises a layer of hydrogel with a thickness between 50 and 2000 µm.

In some embodiments of the second apparatus, the at least one layer of conductive material comprises a conductive adhesive. Optionally, in these embodiments, the conductive adhesive comprises an adhesive polymer and carbon powder, particles, fibers, flakes or nanotubes. Optionally, in these embodiments, the conductive adhesive has a thickness between 10 and 2,000 µm.

Some embodiments of the second apparatus further comprise a flexible self-adhesive backing configured to support the sheet, the first electrode element, and the at least one layer of conductive material so that the front surface of the at least one layer of conductive material can be positioned against a subject's skin.

In some embodiments of the second apparatus, the sheet has a centroid, and a centroid of the first front face of the first electrode element is positioned less than 3 cm away from the centroid of the sheet. In some embodiments of the second apparatus, the sheet has a centroid and a dimension in a direction parallel to the rear face of the sheet, and a centroid of the first front face of the first electrode element is positioned away from the centroid of the sheet by less than 10% of the dimension.

Some embodiments of the second apparatus further comprise a lead that is electrically connected to the first electrode element.

Another aspect of the invention is directed to a second method of applying an alternating electric field to a target region in a subject's body. The second method comprises positioning a first electrode assembly at a first position on or in the subject's body. The first electrode assembly includes a first sheet of anisotropic material having a first front face and a first rear face. The first sheet has a first resistance in a direction that is perpendicular to the first front face. Resistance of the first sheet in directions that are parallel to the first front face is less than half of the first resistance. The first electrode assembly is positioned so that the first front face of the first sheet faces the target region. The second method also comprises positioning a second electrode assembly at a second position on or in the subject's body. The second electrode assembly includes a second sheet of anisotropic material having a second front face and a second rear face. The second sheet has a second resistance in a direction that is perpendicular to the second front face. Resistance of the second sheet in directions that are parallel to the second front face is less than half of the second resistance. The second electrode assembly is positioned so that the second front face of the second sheet faces the target region. The second method also comprises applying an alternating voltage between the first electrode assembly and the second electrode assembly. The applying is performed after positioning the first electrode assembly and the second electrode assembly.

In some instances of the second method, the applying is implemented by applying the alternating voltage between (i) a first electrode element disposed in electrical contact with the first rear face and (ii) a second electrode element disposed in electrical contact with the second rear face.

Some instances of the second method further comprise measuring a first temperature of the first electrode element; measuring a second temperature of the second electrode element; and controlling the applying based on the first temperature and the second temperature.

In some instances of the second method, the first electrode assembly further includes a first layer of conductive material disposed on the first front face. The second electrode assembly further includes a second layer of conductive material disposed on the second front face.

In some instances of the second method, each of the first and second sheets of anisotropic material is a sheet of graphite. In some instances of the second method, each of the first and second sheets of anisotropic material is a sheet of pyrolytic graphite, graphite foil made from compressed high purity exfoliated mineral graphite, or graphitized polymer film. In some instances of the second method, each of the first and second sheets of anisotropic material is nonmetallic.

In some instances of the second method, the resistance of the first sheet in directions that are parallel to the first front face is less than 10% of the first resistance. And the resistance of the second sheet in directions that are parallel to the second front face is less than 10% of the second resistance.

In some instances of the second method, the first sheet has a first thermal conductivity in a direction that is perpendicular to the first front face, wherein thermal conductivity of the first sheet in directions that are parallel to the first front face is more than two times higher than the first thermal conductivity. And the second sheet has a second thermal conductivity in a direction that is perpendicular to the second front face, wherein thermal conductivity of the second sheet in directions that are parallel to the second front face is more than two times higher than the second thermal conductivity.

Another aspect of the invention is directed to a third method of treating a condition in a subject by applying an alternating electric field to a target region in the subject's body. The third method comprises positioning a first electrode assembly at a first position on or in the subject's body. The first electrode assembly includes a first sheet of conductive anisotropic material that has a first front face and a first rear face. The first sheet has a first thermal conductivity in a direction that is perpendicular to the first front face, and the thermal conductivity of the first sheet in directions that are parallel to the first front face is more than two times higher than the first thermal conductivity. The first electrode assembly is positioned so that the first front face of the first sheet faces the target region. The third method also comprises positioning a second electrode assembly at a second position on or in the subject's body. The second electrode assembly includes a second sheet of conductive anisotropic material that has a second front face and a second rear face. The second sheet has a second thermal conductivity in a direction that is perpendicular to the second front face, and the thermal conductivity of the second sheet in directions that are parallel to the second front face is more than two times higher than the second thermal conductivity. The second electrode assembly is positioned so that the second front face of the second sheet faces the target region. And the third method also comprises applying an alternating voltage between the first electrode assembly and the second electrode assembly. The applying is performed after positioning the first electrode assembly and the second electrode assembly.

In some instances of the third method, the applying is implemented by applying the alternating voltage between (i) a first electrode element disposed in electrical contact with the first rear face and (ii) a second electrode element disposed in electrical contact with the second rear face. In some instances of the third method, the target region is in a region selected from one of a torso, brain, and abdomen of the subject.

In some instances of the third method, the condition is a cancer. Optionally, in these instances, the cancer is selected from glioblastoma multiforme, lung cancer, malignant pleural mesothelioma, non-small cell lung cancer, brain metastases, breast cancer, ovarian cancer, prostate cancer, pancreatic cancer, gastric cancer, gastroesophageal junction adenocarcinoma, gastric adenocarcinoma, intestinal cancer, colon cancer, rectal cancer, hepatocellular cancer, uveal cancer and liver cancer.

In some instances of the third method, the condition is a cancer, and the method further comprises administering one or more anti-cancer drugs. Optionally, in these instances, the one or more of the anti-cancer drugs is selected from temozolomide, pemetrexed, cisplatin, carboplatin, paclitaxel, nab-paclitaxel, doxorubicin, cyclophosphamide , trastuzumab, atezolizumab, gemcitabine, mebendazole, sorafenib, oxaliplatin, capecitabin, fluorouracil, Leucovorin, depatuxizumab mafodotin and ABT-751.

In some instances of the third method, the condition is a cancer, and the method further comprises administration of an immune checkpoint inhibitor. Optionally, in these instances, the immune checkpoint inhibitor is selected from one or more of pembrolizumab, nivolumab, cemiplimab, atezolimumab, durvalumab, avelumab, and ipilimumab.

In some instances of the third method, the condition is a cancer, the cancer is a glioblastoma multiforme, and the method further comprises the administration of temozolomide.

In some instances of the third method, the condition is a cancer, and the method further comprises administration of radiation therapy. Optionally, these instances may further comprise administration of one or more of an E2F inhibitor, a CDK4/6 inhibitor and a PARP inhibitor.

Some instances of the third method further comprise administering one or more of an E2F inhibitor, a CDK4/6 inhibitor and a PARP inhibitor.

In some instances of the third method, the condition is a neurodegenerative disease. Optionally, in these instances, the neurodegenerative disease is selected from amyotrophic lateral sclerosis (ALS), Parkinson's disease, Alzheimer's disease, Huntington's disease, neurofibromatosis, multiple sclerosis, or dementia.

In some instances of the third method, the condition is an autoimmune disease. Optionally, in these instances, the autoimmune disease is selected from Rasmussen encephalitis (RE), lupus nephritis, type 1 diabetes, rheumatoid arthritis, and polymyositis.

Some instances of the third method further comprise measuring a first temperature of the first electrode element; measuring a second temperature of the second electrode element; and controlling the applying based on the first temperature and the second temperature.

In some instances of the third method, the first electrode assembly further includes a first layer of conductive material disposed on the first front face, and the second electrode assembly further includes a second layer of conductive material disposed on the second front face.

In some instances of the third method, each of the first and second sheets of anisotropic material is a sheet of pyrolytic graphite. In some instances of the third method, each of the first and second sheets of anisotropic material is graphite foil made from compressed high purity exfoliated mineral graphite or graphitized polymer film.

In some instances of the third method, the thermal conductivity of the first sheet in directions that are parallel to the first front face is more than ten times higher than the first thermal conductivity, and the thermal conductivity of the second sheet in directions that are parallel to the second front face is more than ten times higher than the second thermal conductivity.

In some instances of the third method, the first sheet has a resistance in directions that are parallel to the first front face that is less than 1/2 of the resistance of the first sheet in directions that are perpendicular to the first front face, and the second sheet has a resistance in directions that are parallel to the second front face that is less than 1/2 of the resistance of the second sheet in directions that are perpendicular to the second front face.

In some instances of the third method, the first sheet has a resistance in directions that are parallel to the first front face that is less than 10% of the resistance of the first sheet in directions that are perpendicular to the first front face, and the second sheet has a resistance in directions that are parallel to the second front face that is less than 10% of the resistance of the second sheet in directions that are perpendicular to the second front face.

Another aspect of the invention is directed to a chemotherapeutic drug selected from one or more of temozolomide, pemetrexed, cisplatin, carboplatin, paclitaxel, nab-paclitaxel, doxorubicin, cyclophosphamide, trastuzumab, atezolizumab, gemcitabine, mebendazole, sorafenib, oxaliplatin, capecitabin, fluorouracil, Leucovorin, depatuxizumab mafodotin, and ABT-751, for use in treatment of a cancer selected from glioblastoma multiforme, lung cancer, malignant pleural mesothelioma, non-small cell lung cancer, brain metastases, breast cancer, ovarian cancer, prostate cancer, pancreatic cancer, gastric cancer, gastroesophageal junction adenocarcinoma, gastric adenocarcinoma intestinal cancer, colon cancer, rectal cancer, liver cancer, hepatocellular cancer, and uveal cancer. The chemotherapeutic drug is administered in combination with application of an alternating electric field having a frequency of 50 kHz to 1 MHz using electrode assemblies that include a sheet of anisotropic material.

Optionally, the application of the alternating electric field is implemented by applying an alternating voltage between (i) a first electrode assembly that includes a first sheet of anisotropic material and (ii) a second electrode assembly that includes a second sheet of anisotropic material.

Optionally, the chemotherapeutic drug is administered in further combination with administration of an immune checkpoint inhibitor. Optionally, the chemotherapeutic drug is administered in further combination with administration of an immune checkpoint inhibitor selected from one or more of pembrolizumab, nivolumab, cemiplimab, atezolimumab, durvalumab, avelumab, and ipilimumab.

Optionally, the cancer is a glioblastoma multiforme, and the chemotherapeutic drug comprises temozolomide. Optionally, the cancer is a glioblastoma multiforme, and the chemotherapeutic drug comprises temozolomide and is administered in further combination with administration of an immune checkpoint inhibitor. Optionally, the cancer is a glioblastoma multiforme, and the chemotherapeutic drug comprises temozolomide and is administered in further combination with pembrolizumab.

Optionally, the chemotherapeutic drug is administered in further combination with radiation therapy.

Optionally, the chemotherapeutic drug is administered in further combination with one or more of an E2F inhibitor, a CDK4/6 inhibitor and a PARP inhibitor.

Optionally, the chemotherapeutic drug is administered in combination with application of an alternating electric field having a frequency of 50 kHz to 1 MHz using electrode assemblies that include a sheet of graphite.

Optionally, the chemotherapeutic drug is administered in combination with application of an alternating electric field having a frequency of 50 kHz to 1 MHz using electrode assemblies that include pyrolytic graphite, or graphite foil sheets made from compressed high purity exfoliated mineral graphite, or graphitized polymer film.

Optionally, the application of the alternating electric field is implemented by applying an alternating voltage between (i) a first electrode assembly that includes a first sheet of anisotropic material having a first front face and a first rear face and (ii) a second electrode assembly that includes a second sheet of anisotropic material having a second front face and a second rear face. The thermal conductivity of the first sheet in directions that are parallel to the first front face is more than two times higher than thermal conductivity in directions that are perpendicular to the first front face, and the thermal conductivity of the second sheet in directions that are parallel to the second front face is more than two times higher than the thermal conductivity in directions that are perpendicular to the second front face.

Optionally, the application of the alternating electric field is implemented by applying an alternating voltage between (i) a first electrode assembly that includes a first sheet of anisotropic material having a first front face and a first rear face and (ii) a second electrode assembly that includes a second sheet of anisotropic material having a second front face and a second rear face. The thermal conductivity of the first sheet in directions that are parallel to the first front face is more than ten times higher than thermal conductivity in directions that are perpendicular to the first front face, and the thermal conductivity of the second sheet in directions that are parallel to the second front face is more than ten times higher than thermal conductivity in directions that are perpendicular to the second front face.

Optionally, the application of the alternating electric field is implemented by applying an alternating voltage between (i) a first electrode assembly that includes a first sheet of anisotropic material having a first front face and a first rear face and (ii) a second electrode assembly that includes a second sheet of anisotropic material having a second front face and a second rear face. The first sheet has a resistance in directions that are parallel to the first front face that is less than 1/2 of the resistance of the first sheet in directions that are perpendicular to the first front face, and the second sheet has a resistance in directions that are parallel to the second front face that is less than 1/2 of the resistance of the second sheet in directions that are perpendicular to the second front face.

Optionally, the application of the alternating electric field is implemented by applying an alternating voltage between (i) a first electrode assembly that includes a first sheet of anisotropic material having a first front face and a first rear face and (ii) a second electrode assembly that includes a second sheet of anisotropic material having a second front face and a second rear face. The first sheet has a resistance in directions that are parallel to the first front face that is less than 10% of the resistance of the first sheet in directions that are perpendicular to the first front face, and the second sheet has a resistance in directions that are parallel to the second front face that is less than 10% of the resistance of the second sheet in directions that are perpendicular to the second front face.

Another aspect of the invention is directed to an immune checkpoint inhibitor selected from one or more of pembrolizumab, nivolumab, cemiplimab, atezolimumab, durvalumab, avelumab, and ipilimumab for use in treatment of a cancer selected from glioblastoma multiforme, lung cancer, malignant pleural mesothelioma, non-small cell lung cancer, brain metastases, breast cancer, ovarian cancer, prostate cancer, pancreatic cancer, gastric cancer, gastroesophageal junction adenocarcinoma, gastric adenocarcinoma, intestinal cancer, colon cancer, rectal cancer, liver cancer, hepatocellular cancer, and uveal cancer. The immune checkpoint inhibitor is administered in combination with application of an alternating electric field having a frequency of 50 kHz to 1 MHz using electrode assemblies that include a sheet of anisotropic material.

Another aspect of the invention is directed to an E2F inhibitor, CDK4/6 inhibitor or PARP inhibitor selected from one or more of HLM006474, MRT00033659, YKL-5-124-TFA, YKL-5-124, abemaciclib, ribociclib, trilaciclib, ibrance, lerociclib, alvocidib, roniciclib, riviciclib, milciclib, RGB-286638, NSN3106729, PHA-793887, R547, indirubin, NU610 2, bohemine, CDK9-IN-7, CGP60474, purvalanol A, PF-06873600, nimbolide, FN-1501, AG-024322, ON123300, G1T28, G1T38, AMG925, SHR-6390, BPI-1178, BPI-16350, FCN437, birociclib, BEBT-209, Ty-302, TQB-3616, HS-10342, PF-06842874, CS-2002, MM-D37K, CDK4/6-IN-2, SU9516, AT7519, niraparib, olaparib, and rucaparib for use in treatment of a cancer selected from glioblastoma multiforme, lung cancer, malignant pleural mesothelioma, non-small cell lung cancer, brain metastases, breast cancer, ovarian cancer, prostate cancer, pancreatic cancer, gastric cancer, gastroesophageal junction adenocarcinoma, gastric adenocarcinoma, intestinal cancer, colon cancer, rectal cancer, liver cancer, hepatocellular cancer, and uveal cancer. The E2F inhibitor, CDK 4/6 inhibitor or PARP inhibitor is administered in combination with application of an alternating electric field having a frequency of 50 kHz to 1 MHz using electrode assemblies that include a sheet of anisotropic material.

Another aspect of the invention is directed to the use of a chemotherapeutic drug selected from temozolomide, pemetrexed, cisplatin, carboplatin, paclitaxel, nab-paclitaxel, doxorubicin, cyclophosphamide , trastuzumab, atezolizumab, gemcitabine, mebendazole, sorafenib, oxaliplatin, capecitabin, fluorouracil, Leucovorin, depatuxizumab mafodotin, and ABT-751 for use in manufacture of a medicament for treatment of a cancer selected from glioblastoma multiforme, lung cancer, malignant pleural mesothelioma, non-small cell lung cancer, brain metastases, breast cancer, ovarian cancer, prostate cancer, pancreatic cancer, gastric cancer, gastroesophageal junction adenocarcinoma, gastric adenocarcinoma, intestinal cancer, colon cancer, rectal cancer, liver cancer, hepatocellular cancer, and uveal cancer. The chemotherapeutic drug is administered in combination with application of an alternating electric field having a frequency of 50 kHz to 1 MHz using electrode assemblies that include a sheet of anisotropic material.

Another aspect of the invention is directed to the use of an immune checkpoint inhibitor selected from one or more of pembrolizumab, nivolumab, cemiplimab, atezolimumab, durvalumab, avelumab, and ipilimumab for use in manufacture of a medicament for treatment of a cancer selected from glioblastoma multiforme, lung cancer, malignant pleural mesothelioma, non-small cell lung cancer, brain metastases, breast cancer, ovarian cancer, prostate cancer, pancreatic cancer, gastric cancer, gastroesophageal junction adenocarcinoma, gastric adenocarcinoma, intestinal cancer, colon cancer, rectal cancer, liver cancer, hepatocellular cancer, and uveal cancer. The immune checkpoint inhibitor is administered in combination with application of an alternating electric field having a frequency of 50 kHz to 1 MHz using electrode assemblies that include a sheet of anisotropic material.

Another aspect of the invention is directed to the use of an E2F inhibitor, CDK4/6 inhibitor or PARP inhibitor selected from one or more of HLM006474, MRT00033659, YKL-5-124-TFA, YKL-5-124, abemaciclib, ribociclib, trilaciclib, ibrance, lerociclib, alvocidib, roniciclib, riviciclib, milciclib, RGB-286638, NSN3106729, PHA-793887, R547, indirubin, NU610 2, bohemine, CDK9-IN-7, CGP60474, purvalanol A, PF-06873600, nimbolide, FN-1501, AG-024322, ON123300, G1T28, G1T38, AMG925, SHR-6390, BPI-1178, BPI-16350, FCN437, birociclib, BEBT-209, Ty-302, TQB-3616, HS-10342, PF-06842874, CS-2002, MM-D37K, CDK4/6-IN-2, SU9516, AT7519, niraparib, olaparib, and rucaparib in the manufacture of a medicament for use in treatment of a cancer selected from glioblastoma multiforme, lung cancer, malignant pleural mesothelioma, non-small cell lung cancer, brain metastases, breast cancer, ovarian cancer, prostate cancer, pancreatic cancer, gastric cancer, gastroesophageal junction adenocarcinoma, gastric adenocarcinoma, intestinal cancer, colon cancer, rectal cancer, liver cancer, hepatocellular cancer, and uveal cancer. The E2F inhibitor, CDK 4/6 inhibitor or PARP inhibitor is administered in combination with application of an alternating electric field having a frequency of 50 kHz to 1 MHz using electrode assemblies that include a sheet of anisotropic material. Optionally, the E2F inhibitor, CDK4/6 inhibitor or PARP inhibitor is further administered in combination with radiation therapy.

Another aspect of the invention is directed to a third apparatus for applying an alternating electric field to a target region in a subject's body. The third apparatus comprises a first electrode assembly, second electrode assembly, and an alternating voltage generator. The first electrode assembly includes a first sheet of anisotropic material having a first front face and a first rear face. The first sheet has a first thermal conductivity in a direction that is perpendicular to the first front face, and the thermal conductivity of the first sheet in directions that are parallel to the first front face is more than two times higher than the first thermal conductivity. The second electrode assembly includes a second sheet of anisotropic material having a second front face and a second rear face. The second sheet has a second thermal conductivity in a direction that is perpendicular to the second front face, and the thermal conductivity of the second sheet in directions that are parallel to the second front face is more than two times higher than the second thermal conductivity. The alternating voltage generator is configured to apply an alternating voltage between the first electrode assembly and the second electrode assembly.

In some embodiments of the third apparatus, the thermal conductivity of the first sheet in directions that are parallel to the first front face is more than ten times higher than the first thermal conductivity, and the thermal conductivity of the second sheet in directions that are parallel to the second front face is more than ten times higher than the second thermal conductivity.

In some embodiments of the third apparatus, the first electrode assembly further includes a first layer of conductive material disposed on the first front face, and the second electrode assembly further includes a second layer of conductive material disposed on the second front face.

In some embodiments of the third apparatus, each of the first and second sheets of anisotropic material is a sheet of graphite. In some embodiments of the third apparatus, each of the first and second sheets of anisotropic material is a sheet of pyrolytic graphite, graphite foil made from compressed high purity exfoliated mineral graphite, or graphitized polymer film.

Another aspect of the invention is directed to a fourth apparatus for applying an alternating electric field to a target region in a subject's body. The fourth apparatus comprises a first electrode assembly, a second electrode assembly, and an alternating voltage generator. The first electrode assembly includes a first sheet of anisotropic material having a first front face and a first rear face. The first sheet has a first resistance in a direction that is perpendicular to the first front face, and the resistance of the first sheet in directions that are parallel to the first front face is less than half of the first resistance. The second electrode assembly includes a second sheet of anisotropic material having a second front face and a second rear face. The second sheet has a second resistance in a direction that is perpendicular to the second front face, and the resistance of the second sheet in directions that are parallel to the second front face is less than half of the second resistance. The alternating voltage generator is configured to apply an alternating voltage between the first electrode assembly and the second electrode assembly.

In some embodiments of the fourth apparatus, the resistance of the first sheet in directions that are parallel to the first front face is less than 10% of the first resistance, and the resistance of the second sheet in directions that are parallel to the second front face is less than 10% of the second resistance.

In some embodiments of the fourth apparatus, the first electrode assembly further includes a first layer of conductive material disposed on the first front face, and the second electrode assembly further includes a second layer of conductive material disposed on the second front face.

In some embodiments of the fourth apparatus, each of the first and second sheets of anisotropic material is a sheet of graphite. In some embodiments of the fourth apparatus, each of the first and second sheets of anisotropic material is a sheet of pyrolytic graphite, graphite foil made from compressed high purity exfoliated mineral graphite, or graphitized polymer film.

### BRIEF DESCRIPTION OF THE DRAWINGS

FIG. 1A is a schematic representation of a prior art electrode assembly.
FIG. 1B is a cross sectional view of electrode elements of the prior art electrode assembly, taken along the dashed line in FIG. 1A.
FIG. 1C is a cross sectional view showing the heat generation properties of a prior art electrode element.
FIG. 1D is a cross sectional view showing the heat generation properties of a hypothetical modification to the FIG. 1B electrode element.
FIG. 2 is a plan schematic representation of an electrode assembly including electrode elements that is used for applying TTFields to a subject's body.
FIG. 3A is a cross sectional representation of a first embodiment including electrode elements E1, E2, taken along the dashed line in FIG. 2.
FIG. 3B is a cross sectional view showing the heat generation properties of the FIG. 3A embodiment.
FIG. 4A is a thermal image of a prior art electrode assembly.
FIG. 4B is a thermal image of an electrode assembly corresponding to the FIG. 3A embodiment.
FIG. 4C is a graph comparing the thermal properties of the prior art electrode assembly with the FIG. 3A embodiment.
FIG. 4D shows a thermal camera image of simulated electrodes arrays built using metal (aluminum) sheets.
FIG. 4E shows a thermal camera image of simulated electrodes arrays built using sheets of anisotropic material (pyrolytic graphite).
FIG. 4F depicts experimental results when electrode arrays with and without a sheet of graphite were used to apply TTFields to the torso of rats.
FIG. 5 is a cross sectional representation of a second embodiment including electrode elements E1, E2, taken along the dashed line in FIG. 2.
FIG. 6 is a cross sectional representation of a third embodiment that includes a single electrode element E1.
FIG. 7 is a cross sectional representation of a fourth embodiment that includes a single electrode element E1.
FIG. 8 is a cross sectional representation of a fifth embodiment that includes a single electrode element E1.
FIG. 9 is a block diagram of a system incorporating two electrode assemblies that is used for applying TTFields to a subject's body.

Various embodiments are described in detail below with reference to the accompanying drawings, wherein like reference numerals represent like elements.

### DESCRIPTION OF THE PREFERRED EMBODIMENTS

This application describes exemplary electrode assemblies that may be used, e.g., for delivering TTFields to a subject's body and treating one or more cancers or tumors located in the subject's body.

When TTFields are applied to a subject's body, the temperature at the subject's body may increase proportionally to the induced electric field. Regulations limit the amount of current that can be driven through a transducer array to an amount that keeps the measured temperature at locations on the subject's body below a temperature threshold. As practiced in the art, the temperature at the location of the transducer arrays on the subject's body is controlled to be below the temperature threshold by reducing the operational current driven by the transducer arrays and reducing the strength of the resulting TTFields. This in turn becomes an over-riding limitation on the TTFields strength that can be used to treat the tumor. Accordingly, there is a need in the art to safely access higher TTField strengths without exceeding the temperature threshold at the subject's skin.

On transducer arrays that comprise multiple electrode elements, the portions of the transducer arrays positioned directly beneath the electrode elements get hotter than the portions of the transducer arrays positioned between the electrode elements. Furthermore, on transducer arrays that comprise multiple electrode elements, higher currents flow through the electrode elements located along the edge of the array compared to the electrode elements located toward the middle of the array. Further still, an electrode element located at a corner or similar sharp bend in the edge of the array will have a higher current than other electrode elements along the edge and near the center of the array. The tendency of a transducer array to drive higher currents through electrode elements located along the edge of the array, and particularly at the corners, is referred to herein as the "edge effect."

An uneven distribution of current through the transducer array due to either the distribution of the electrode elements or the edge effect can lead to higher temperature zones (or "hot spots") e.g., at the corners or edges of the transducer array. These hot spots are the locations that reach the threshold temperature first and therefore control the requirement to reduce the current. As such, the generation of hot spots limits the maximum operational current that may be driven by a transducer array, and the strength of the resulting TTFields.

The inventors have now recognized that a need exists for transducer arrays that reduce or minimize uneven distribution of current and thereby allow the application of higher operating currents. Transducer arrays operated with increased current can induce stronger TTFields in the subject's body, ultimately leading to better patient outcomes. The electrode assemblies disclosed herein allow current and heat to be spread evenly over the array thereby minimizing or eliminating hot spots.

The embodiments described herein incorporate into the electrode assembly a sheet of material having anisotropic thermal properties and/or anisotropic electrical properties, as described below. If the sheet of material has anisotropic thermal properties, then the sheet spreads the heat out more evenly over a larger surface area. If the sheet of material has anisotropic electrical properties, then the sheet spreads the current out more evenly over a larger surface area. In each case, this lowers the temperature of the hot spots and raises the temperature of the cooler regions when a given AC voltage is applied to the electrode assembly (as compared to the prior art configuration described above). Accordingly, the current can be increased (thereby increasing the therapeutic effect) without exceeding the safety temperature threshold at any point on the subject's skin.

In some embodiments, the anisotropic material is anisotropic with respect to electrical conductivity properties. In some embodiments, the anisotropic material is anisotropic with respect to thermal conductivity properties. In some preferred embodiments, the anisotropic material is anisotropic with respect to both electrical conductivity properties and thermal conductivity properties.

The anisotropic thermal properties include directional thermal properties. Specifically, the sheet of anisotropic material has a first thermal conductivity in a direction that is perpendicular to its front face. And the thermal conductivity of the sheet in directions parallel to the front face is more than two times higher than the first thermal conductivity. In some preferred embodiments, the thermal conductivity in the parallel directions is more than ten times higher than the first thermal conductivity. For example, the thermal conductivity of the sheet in directions that are parallel to the front face may be more than: 1.5 times, 2 times, 3 times, 5 times, 10 times, 20 times, 30 times, 100 times, 200 times, or even more than 1,000 times higher than the first thermal conductivity. In some embodiments, the thermal conductivity of the sheet of anisotropic material in directions that are parallel to the front face is between 5 times and 30 times higher than the first thermal conductivity. In some embodiments, the thermal conductivity of the sheet of anisotropic material in directions that are parallel to the front face is between 1.5 times and 10 times higher than the first thermal conductivity. For example, the thermal conductivity of a sheet of pyrolytic graphite in directions that are in the x-y plane is between 10 times and 20 times higher than its thermal conductivity in the perpendicular z-direction.

The anisotropic electrical properties include directional electrical properties. Specifically, the sheet has a first resistance in a direction that is perpendicular to its front face. And resistance of the sheet in directions parallel to the front face is less than the first resistance. In some preferred embodiments, the resistance in the parallel directions is less than half of the first resistance or less than 10% of the first resistance. For example, the resistance of the sheet 70 in directions that are parallel to the front face may be less than: 75%, 50%, 40%, 30%, 20%, 10%, 5%, 1%, 0.5%, 0.1%, or even less than 0.05% of the first resistance. In some embodiments, the resistance of the sheet of anisotropic material in directions that are parallel to the front face is between 10% and 75% of the first resistance. In some embodiments, the resistance of the sheet of anisotropic material in directions that are parallel to the front face is between 0.05% and 10 % of the first resistance. For example, the electrical resistivity of a sheet of pyrolytic graphite in directions that are in the x-y plane is approximately three orders of magnitude (1,000 times) lower than its electrical resistivity in the perpendicular z-direction.

In some embodiments (e.g., when the sheet of anisotropic material is a sheet of pyrolytic graphite), the sheet of anisotropic material has both anisotropic electrical properties and anisotropic thermal properties.

In some embodiments (e.g., when the sheet of anisotropic material is a sheet of pyrolytic graphite), the sheet of anisotropic material is nonmetallic. These embodiments are particularly advantageous in situations where preventing the transfer of ions into a subject's body is desirable. More specifically, using a metallic sheet could result in the transfer of metal ions into a subject's body. In situations where this is not desirable, embodiments that use nonmetallic sheets of anisotropic material are preferable.

The present invention can be understood more readily by reference to the following detailed description, examples, drawings, and claims, and their previous and following description. However, it is to be understood that this invention is not limited to the specific apparatuses, devices, systems, and/or methods disclosed unless otherwise specified, and as such, of course, can vary.

Headings are provided for convenience only and are not to be construed to limit the invention in any manner. Embodiments illustrated under any heading or in any portion of the disclosure may be combined with embodiments illustrated under the same or any other heading or other portion of the disclosure.

Any combination of the elements described herein in all possible variations thereof is encompassed by the invention unless otherwise indicated herein or otherwise clearly contradicted by context.

As used in the specification and the appended claims, the singular forms "a," "an" and "the" include plural referents unless the context clearly dictates otherwise.

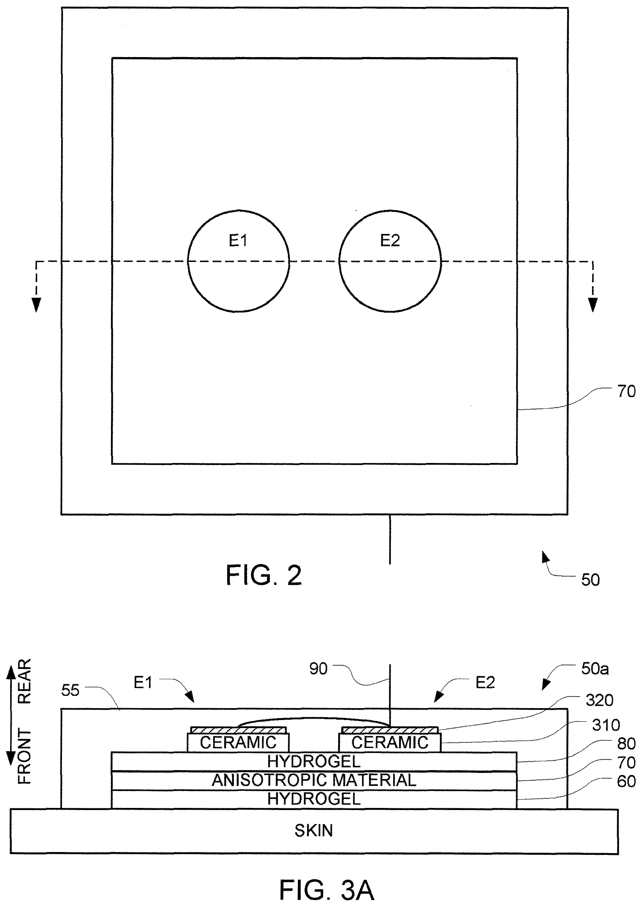

FIG. 2 is a schematic representation of an electrode assembly 50 of an embodiment including electrode elements used for applying TTFields to a subject's body. In FIG. 2, only two electrode elements labeled E1 and E2 are shown, but additional electrode elements may be included in the electrode assembly 50. In alternative embodiments, the electrode assembly 50 includes only a single electrode element. Notably, FIG. 2 depicts an electrode assembly 50 generically, and those electrode assemblies E1 and E2 can have different configurations (e.g., as described below in connection with FIGS. 3A-8).

FIG. 3A is a cross sectional representation of a first embodiment of an electrode assembly 50a including electrode elements E1, E2, taken along the dashed line in FIG. 2.

In the FIG. 3A embodiment, the electrode assembly 50a includes a sheet of anisotropic material 70 having a front face (facing towards the subject's skin in FIG. 3A) and a rear face. This sheet 70 has a first thermal conductivity in a direction that is perpendicular to the front face. Thermal conductivity of the sheet 70 in directions that are parallel to the front face is more than two times higher than the first thermal conductivity. In some preferred embodiments, the thermal conductivity of the sheet 70 in directions that are parallel to the front face is more than ten times higher than the first thermal conductivity. The sheet 70 in the FIG. 3A embodiment is also anisotropic in another respect. More specifically, the sheet 70 has a first resistance in a direction that is perpendicular to the front face, and the resistance of the sheet in directions that are parallel to the front face is less than half of the first resistance. In some embodiments, the resistance of the sheet in directions that are parallel to the front face is less than 10% of the first resistance.

The electrode assembly 50a includes a sheet of conductive anisotropic material 70 having a front face (facing towards the subject's skin in FIG. 3A) and a rear face. The sheet of conductive anisotropic material 70 could be a sheet of graphite. Examples of suitable forms of graphite include synthetic graphite, such as pyrolytic graphite (including, but not limited to, Pyrolytic Graphite Sheet (PGS), available from Panasonic Industry, Kadoma, Osaka, Japan), other forms of synthetic graphite, including but not limited to, graphite foil made from compressed high purity exfoliated mineral graphite (including, but not limited to, that supplied by MinGraph^{®} 2010A Flexible Graphite, available from Mineral Seal Corp., Tucson, Arizona, USA), or graphitized polymer film, e.g., graphitized polyimide film, (including, but not limited to, that supplied by Kaneka Corp., Moka, Tochigi, Japan. In alternative embodiments, conductive anisotropic materials other than graphite may be used instead of graphite.

In some embodiments, the sheet of anisotropic material 70 is a sheet of pyrolytic graphite. Thermal conductivity of pyrolytic graphite sheets in directions that are parallel to the front face of those sheets (i.e., in the x-y plane) is typically more than 50 times higher than the thermal conductivity of those sheets in directions that are perpendicular to the front face (i.e., in the z direction). And electrical resistivity of pyrolytic graphite sheets in directions that are parallel to the front face of those sheets (i.e., in the x-y plane) is typically less than 2% of the electrical resistivity of those sheets in directions that are perpendicular to the front face (i.e., in the z direction).

In other embodiments, the sheet of anisotropic material 70 is graphite foil made from compressed high purity exfoliated mineral graphite. In other embodiments, the anisotropic material may be pyrolytic carbon, or graphitized polymer film. In other embodiments, the anisotropic material may be boron nitride. Other embodiments may utilize sheets of other conducting materials with anisotropic properties. In some embodiments (e.g., when the sheet of anisotropic material is a sheet of a synthetic graphite, such as pyrolytic graphite or the compressed high purity exfoliated mineral graphite or graphitized polymer film), the sheet of anisotropic material 70 is nonmetallic.

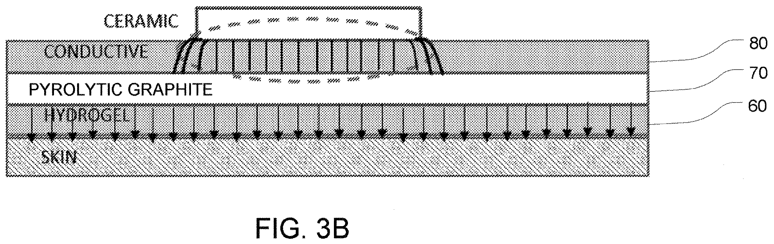

The electrode assembly 50a further includes at least one layer of conductive material 60 disposed on the front face of the sheet 70, and the at least one layer of conductive material 60 has a biocompatible front surface. Note that the embodiment illustrated in FIG. 3, there is only a single layer of conductive material 60, and that single layer is biocompatible. But in alternative embodiments (not shown) there could be more than one layer, in which case only the front layer must be biocompatible. The at least one layer of material 60 is configured to ensure good electrical contact between the device and the body. In some embodiments, the at least one layer of material 60 should cover the entire front face of the sheet of anisotropic material 70. The at least one layer of material 60 may be the same size or larger than the sheet of anisotropic material 70. In some embodiments (and as shown in FIG. 3A), the at least one layer of conductive material 60 comprises a single layer of hydrogel. In these embodiments, the hydrogel may have a thickness between 50 and 2000 µm, such as, from 100 to 1000 µm, or even 300 to 500 µm. In some embodiments, the at least one layer of conductive material 60 is a single layer of non-hydrogel biocompatible conductive adhesive. In some embodiments, the at least one layer of conductive material 60 is a single layer of non-hydrogel biocompatible conductive adhesive such as the developmental product FLX068983 - FLEXcon^{®} OMNI-WAVE^{™} TT 200 BLACK H-502 150 POLY H-9 44PP-8 from FLEXcon, Spencer, MA, USA, or other such OMNI-WAVE products from FLEXcon; or ARcare^{®} 8006 electrically conductive adhesive composition manufactured and sold by Adhesives Research, Inc. (Glen Rock, PA, USA). Non-hydrogel conductive adhesives may comprise a waterless polymer with adhesive properties and carbon particles, powder, fibers, flakes or nanotubes. The adhesive polymer may be, for example, an acrylic polymer or a silicone polymer, or combination thereof, which may be available as acrylic- or silicone-based carbon-filled adhesive tapes. The adhesive may additionally include one or more conductive polymer (such as, for example, polyaniline (PANI), or poly(3,4-ethylenedioxythiophene (PEDOT), or others known in the art). The conductive filler in the at least one layer of conductive material 60 should be non-metallic. In these embodiments, the biocompatible conductive adhesive may have a thickness between 10 and 2,000 µm, such as, from 20 to 1000 µm, or even 30 to 400 µm.

The electrode assembly 50a further includes a first electrode element E1 positioned behind the sheet 70. The first electrode element E1 has a first front face disposed in electrical contact with the rear face of the sheet 70. In the FIG. 3A embodiment, the first electrode element E1 includes a first layer of dielectric (e.g., ceramic) material 310 having a front face and a rear face, and a first layer of metal 320 disposed on the rear face of the first layer of dielectric material 310. The front face of the first layer of dielectric material 310 is the first front face of the first electrode element E1. Note that while the figures (e.g., FIG. 3A) depict the dielectric material 310 as "ceramic," a variety of other suitable dielectric materials may be used instead of ceramic materials. Examples include a polymer layer that has a dielectric constant of at least 10, or another material having a dielectric constant of at least 10.

In some embodiments, the layer of dielectric material 310 can have a dielectric constant ranging from 10 to 50,000. In some embodiments, the layer of dielectric material 310 comprises a high dielectric polymer material such as poly(vinylidene fluoride-trifluoroethylene-chlorotrifluoroethylene) and/or poly(vinylidene fluoride-trifluoroethylene-1-chlorofluoroethylene). Those two polymers are abbreviated herein as "Poly(VDF-TrFE-CTFE)" and "Poly(VDF-TrFE-CFE)," respectively. These embodiments are particularly advantageous because the dielectric constant of these materials is on the order of 40. In some embodiments, the polymer layer can be poly(vinylidene fluoride-trifluoroethylene-chlorotrifluoroethylene-chlorofluoroethylene) or "Poly(VDF-TrFE-CTFE-CFE)."

In some embodiments, the layer of dielectric material 310 comprises a terpolymer comprising polymerized units of monomers such as VDF, TrFE, CFE and/or CTFE in any suitable molar ratio. Suitable terpolymers include those, for example, having 30 to 80 mol% VDF, 5 to 60 mol% TrFE, with CFE and/or CTFE constituting the balance of the mol% of the terpolymer.

In some embodiments, the sheet 70 has a centroid, and the centroid of the first front face of the first electrode element E1 is positioned less than 3 cm away from the centroid of the sheet 70. In some embodiments, the sheet 70 has a centroid and a dimension parallel to the rear face of the sheet 70 (e.g., a length or a width), and the centroid of the first front face of the first electrode element E1 is positioned away from the centroid of the sheet 70 by less than 30%, or by less than 10% of the dimension.

The electrode assembly 50a further includes a first rear layer of conductive material 80 positioned between the first front face of the first electrode element E1 (i.e., the front face of the first layer of dielectric material 310) and the rear face of the sheet 70. The first rear layer of conductive material 80 facilitates the electrical contact between the first front face of the first electrode element E1 and the rear face of the sheet 70. In the illustrated embodiment, the rear layer of conductive material 80 is a layer of hydrogel. But in alternative embodiments, a different conductive material (e.g., conductive grease, conductive adhesive, conductive tape, conductive composite, etc.) could be used. In some embodiments, the conductive material 80 may be a non-hydrogel conductive adhesive, such as described above.

The electrode assembly 50a may optionally include one or more additional electrode elements. In the illustrated embodiment, the electrode assembly 50a includes a second electrode element E2 positioned behind the sheet 70. The second electrode element E2 has a second front face disposed in electrical contact with the rear face of the sheet 70. The two electrode elements E1, E2 in FIG. 3A have identical structures. Thus, the second electrode element E2 includes a second layer of dielectric (e.g., ceramic) material 310 having a front face and a rear face, and a second layer of metal 320 disposed on the rear face of the second layer of dielectric material 310. The front face of the second layer of dielectric material 310 is the second front face of the second electrode element E2. In some embodiments, the collective area of all the electrode elements is less than the area of the sheet 70, less than half the area of the sheet 70, less than one quarter the area of the sheet 70, or less than one tenth the area of the sheet 70.

The first rear layer of conductive material 80 is positioned between the second front face of the second electrode element E2 (i.e., the front face of the second layer of dielectric material 310) and the rear face of the sheet 70. The first rear layer of conductive material 80 facilitates the electrical contact between the second front face of the second electrode element E2 and the rear face of the sheet 70. As described for E1, and as shown in FIG. 3A, the conductive material 80 may be a layer of hydrogel, but in alternative embodiments, a different conductive material may be used (e.g., conductive grease, conductive adhesive including the non-hydrogel conductive adhesive described above, conductive tape, conductive composite, etc.).

The metal layers 320 of all of the electrode elements (i.e., E1 and E2 in the illustrated embodiment), may be wired together (e.g., using wires, traces on a flex circuit, etc.) to a lead 90. The lead 90 supplies an AC voltage from an AC voltage generator (not shown) to the electrode elements to generate the TTFields when the electrode assembly 50a is affixed to the subject's body for treatment.

Optionally, the electrode assembly 50a includes a flexible self-adhesive backing 55 configured to support the sheet 70, the first electrode element E1 (and any other electrode elements present in the electrode assembly), and the at least one layer of conductive material 60 so that the front surface of the at least one layer of conductive material 60 can be positioned against the subject's skin.

As noted above, FIG. 2 is a plan schematic representation of an electrode assembly 50 including electrode elements E1, E2. This view of FIG. 2 (not to scale) also demonstrates that the area of the sheet 70 is larger (e.g., at least 2 times larger, at least 4 times larger, or at least 10 times larger) than the combined areas of the electrode elements E1, E2. When an AC voltage is applied to the electrode elements E1, E2, heat spreads out across the entire sheet 70, which minimizes or eliminates hot spots. In addition to spreading and altering the directionality of the heat (minimizing heat in specific locations), the spread has an additional effect in dissipating the heat since the majority is directed in the x-y plane to the edges of the sheet, which terminate in a room temperature (cooler) heat sink, rather than proceeding in the z-direction to a much warmer body temperature. The area therefore cools much faster.

This reduction in hot spots (as compared to the prior art) becomes apparent by comparing FIG. 1C to FIG. 3B. More specifically, FIG. 1C shows the current distribution and heat generation for prior art electrode elements, each of which is positioned on a conductive hydrogel layer that covers about the same area (in the x-y plane) as the electrode element. As shown in FIG. 1C, all the current passes through the hydrogel layer directly beneath the electrode elements, which results in hot spots directly beneath the electrode elements.

One might initially think that this problem could be solved by increasing the area of the hydrogel to cover all the regions between the electrode elements (i.e., by covering a significantly larger area in the x-y plane than that of the electrode elements). But this is not the case. More specifically, FIG. 1D shows the current distribution and heat generation for this hypothetical electrode assembly. As shown in FIG. 1D, all the current still passes through the hydrogel layer directly beneath the electrode elements, which results in hot spots directly beneath the electrode elements.

In contrast, FIG. 3B shows the current distribution for the FIG. 3A embodiment. As shown in FIG. 3B, the current is still distributed in the rear conductive material layer (for example, 80 in FIG. 3B) only in the area below the electrode element. However, the sheet of anisotropic material 70 spreads the heat out across its entire area because the thermal conductivity in the horizontal directions (i.e., in directions parallel to the face of the sheet) is much higher than its thermal conductivity in the vertical direction. In addition to spreading out the heat, the low electrical resistance of the sheet 70 in the horizontal direction spreads the current outward throughout the sheet 70, and this spread-out current distribution continues in the layer of conductive material 60 (hydrogel in both FIG. 3A and FIG. 3B), and thence to the subject's skin. Because the current and heat in this embodiment are both spread out over a larger area of the layer of conductive material 60, hotspots are eliminated (or at least minimized). This means that for a given applied AC voltage, the hottest point beneath the electrode assembly in the FIG. 3A/B embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 3A embodiment. And this increase in current will advantageously increase the efficacy of the TTFields treatment. Similar results can be achieved when the hydrogel is replaced with a conductive adhesive composite.

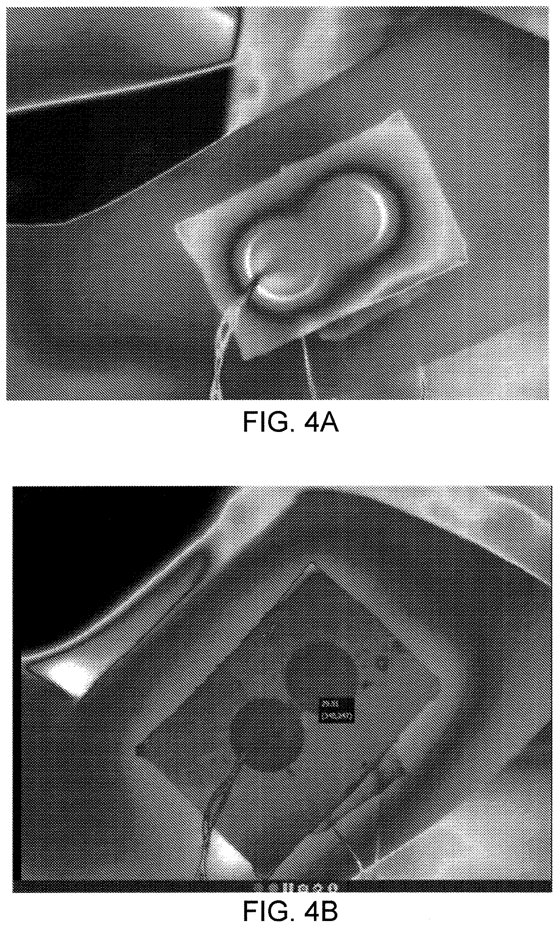

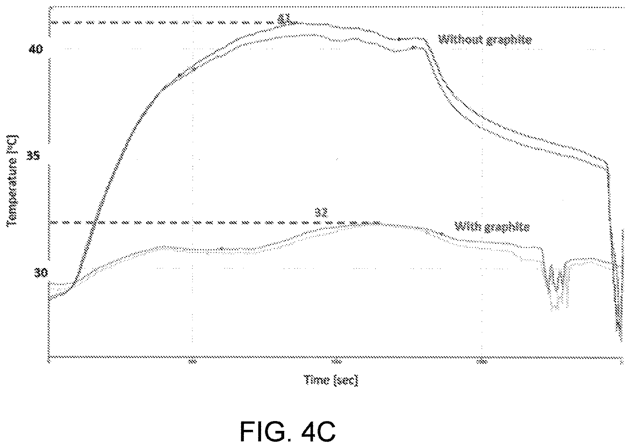

The superior performance of the FIG. 3A embodiment is demonstrated in FIGS. 4A, 4B, and 4C. FIG. 4A is a thermal image of a prior art electrode assembly that includes two electrode elements and a layer of hydrogel disposed on the front faces of the electrode elements (see, e.g., FIG. 1B). There is no sheet of anisotropic material between the front faces of the electrode elements and the rear face of the layer of hydrogel. In use, the front face of the layer of hydrogel is positioned on the subject's skin. FIG. 4A shows hot spots generated in the areas that correspond to the electrode elements.

FIG. 4B is a thermal image of an electrode assembly corresponding to the FIG. 3A embodiment where pyrolytic graphite was used as the anisotropic material. FIG. 4B shows that hot spots such as those generated in the prior art electrode assembly have been minimized, and also that the maximum temperature has been reduced. FIG. 4C is a graph comparing the thermal performance of the FIG. 3A embodiment (utilizing pyrolytic graphite as the anisotropic material) with the prior art (no anisotropic material) for the same applied current (500 mA). Notably, the hottest portion of the prior art electrode assembly was 41° C. But when the same 500 mA current was applied to the FIG. 3A embodiment, the hottest portion of the electrode assembly was only 32° C. Similar experiments were performed utilizing graphite foil made from compressed high purity exfoliated mineral graphite as the anisotropic material, with similar results.

In a related experiment, optimized conventional arrays (no anisotropic material), running with 2 A applied current, ran up to the maximum 40 °C average temperature, and were thereby limited. The same type of array with an added pyrolytic graphite sheet (in the manner of the FIG. 3A embodiment) was able to run at an increased power level (with 3 A applied current), and ran at 38 °C average temperature, 2-3 °C below the temperature threshold limit. This result suggests that the inventive apparatus and methods described herein should be able to achieve more beneficial treatment results by operating at higher applied currents.

An experimental simulation of electrodes for treating a target location in a body compared the heat distribution obtained using pyrolytic graphite sheets to the heat distribution obtained using metal sheets. In one half of the experiment, a phantom gel was placed sandwiched between two sheets of metal (aluminum) and a voltage was applied (directly to the center of the sheet) between the two metal sheets. In the other half of the experiment, a phantom gel was placed sandwiched between two sheets of pyrolytic graphite and a voltage was applied (directly to the center of the sheet) between the two pyrolytic graphite sheets. Metals are generally excellent conductors (both thermal conductivity and electrical conductivity). Pyrolytic graphite sheets also have good electrical conductivity (and higher electrical conductivity in the x-y plane than in the perpendicular z-direction). However, the electrical conductivity in x-y plane for pyrolytic graphite is two orders of magnitude smaller than that of the conventional metals. When voltage is applied between a pair of metal sheets (aluminum), the high conductivity and equipotential properties of aluminum sheet result in a higher current density at the edges of the sheet resulting in an unequal heating of different areas. In contrast, applying voltage between two graphite sheets beneficially results in much more uniform current densities at the sheet center and edges (due to the higher resistance in the x-y plane compared to metal), and results in a more uniform temperature profile of the sheet.

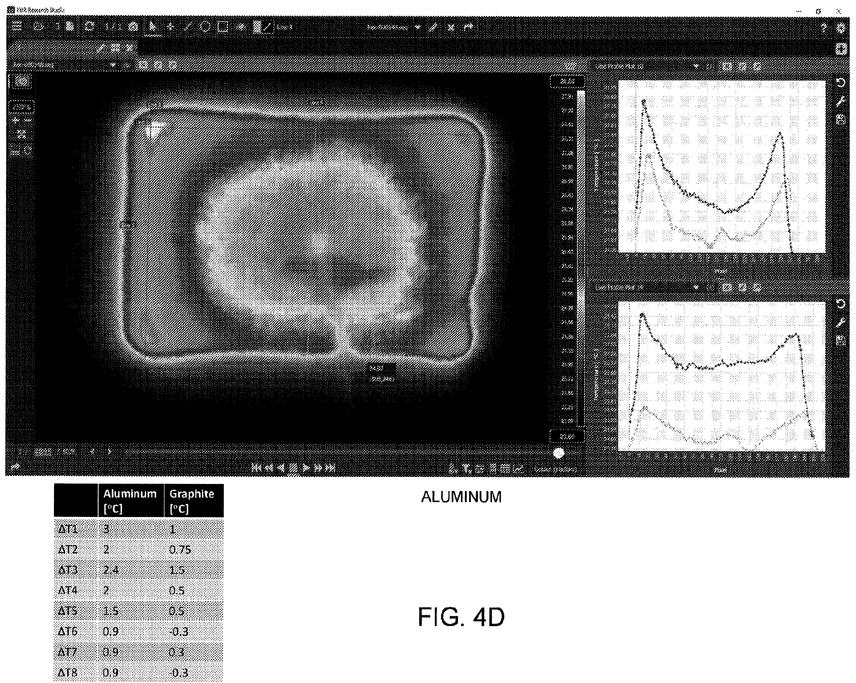

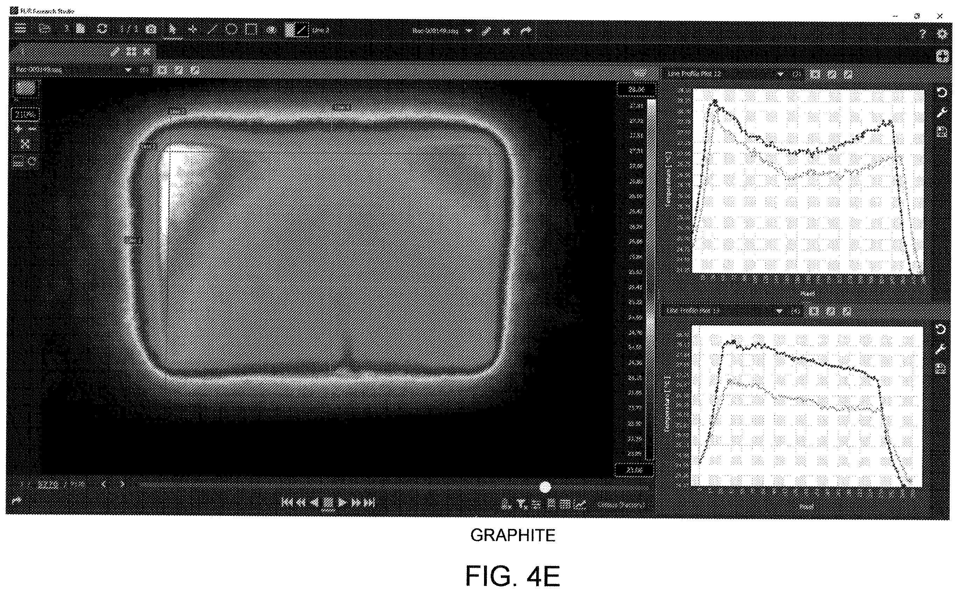

FIGS. 4D and 4E respectively show thermal camera images of the simulated electrodes arrays built using metal (aluminum) sheets and the simulated electrode arrays built using sheets of anisotropic material (pyrolytic graphite). The aluminum sheet results in an uneven heat distribution map which causes the outer edges to reach the threshold temperature first and therefore control the requirement to reduce the current. In contrast, the sheet of pyrolytic graphite produces a very even heat distribution across the entire sheet.

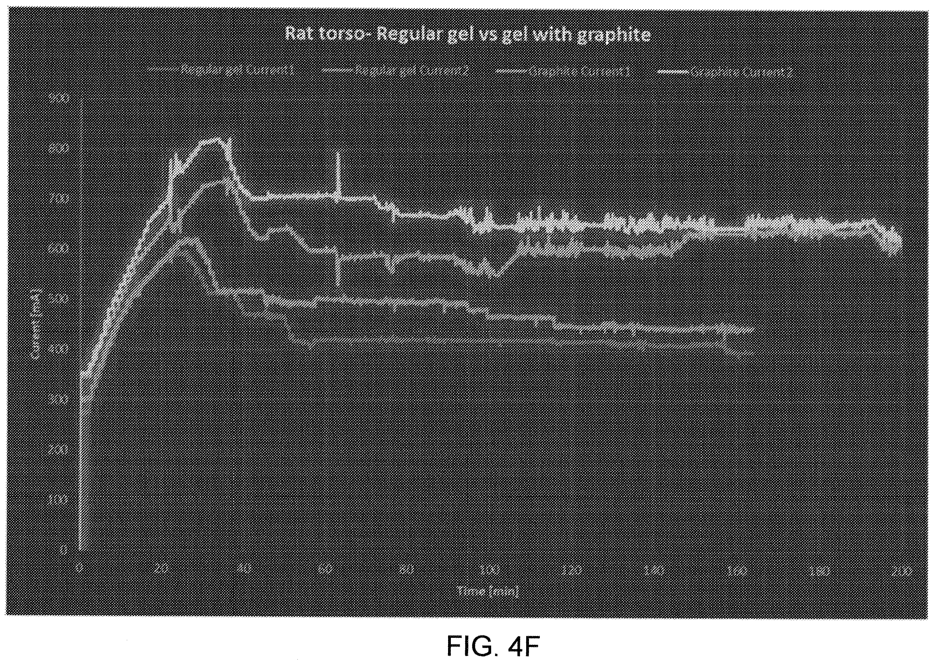

FIG. 4F depicts experimental results when electrode arrays with and without a sheet of graphite were used to apply TTFields to the torso of rats (using small animal arrays). The two lower traces show the measured current for two rats when the prior art electrode arrays depicted in FIG. 1A/1B were used, while the two upper traces show the measured current for two rats when the electrode elements depicted in FIG. 3A were used (using a sheet of graphite as the layer of anisotropic material). The thermal setpoint was identical for all runs. Notably, when the sheet of graphite was included, the improved heat and current distribution attributable to the graphite resulted in resistances that were 20% lower and currents that were 50% higher for the same thermal setpoint. And because higher currents are associated with improved outcomes, these experiments show that incorporating a layer of anisotropic material into the electrode arrays can provide improved outcomes.

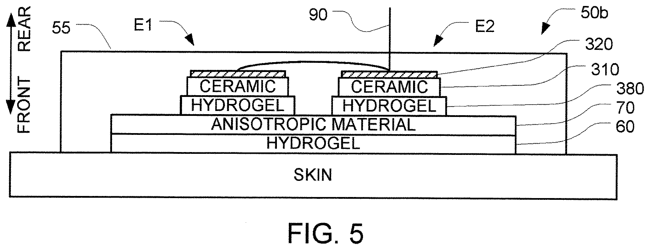

FIG. 5 is a cross sectional representation of a second embodiment of an electrode assembly 50b including electrode elements E1, E2, taken along the dashed line in FIG. 2. The FIG. 5 embodiment is similar to the FIG. 3A embodiment in all respects (including the figure labeling) except as follows. The FIG. 3A embodiment includes a large rear layer of conductive material 80 (e.g., hydrogel) positioned between the sheet 70 and the front faces of both the first and second electrode elements E1 and E2. In contrast, the FIG. 5 embodiment includes a separate region of conductive material 380 for each individual electrode element. Thus, the FIG. 5 embodiment includes a first rear layer of conductive material 380 positioned between the first front face of the first electrode element E1 and the rear face of the sheet 70, and also includes a second rear layer of conductive material 380 positioned between the second front face of the second electrode element E2 and the rear face of the sheet 70. The first and second rear layers of conductive material 380 facilitate the electrical contact between the respective electrode front faces and the rear face of the sheet 70. In the illustrated embodiment, the rear layers of conductive material 380 are layers of hydrogel. But in alternative embodiments, different conductive materials (e.g., conductive grease, conductive adhesive including the non-hydrogel conductive adhesives discussed above, conductive tape, conductive composite, etc.) could be used. In some embodiments, the collective area of all the electrode elements is less than the area of the sheet 70, less than half the area of the sheet 70, less than one quarter the area of the sheet 70, or less than one tenth the area of the sheet 70.

As in the FIG. 3A embodiment, the current in the FIG. 5 embodiment is still concentrated in the rear layers of conductive material 380 only in the areas below the electrode elements. The sheet of anisotropic material 70 spreads out the heat and the current as described above in connection with the FIG. 3A embodiment, which eliminates or at least minimizes hot spots. This means that for a given applied AC voltage, the hottest point beneath the electrode assembly in the FIG. 5 embodiment will be at a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 5 embodiment. And this increase in current will advantageously increase the efficacy of the TTFields treatment.

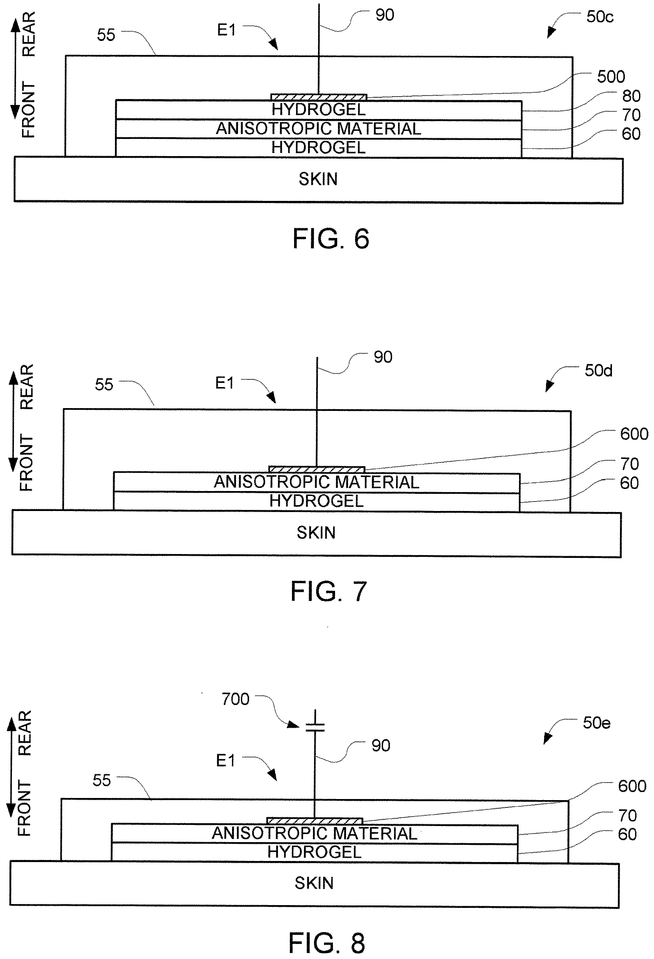

FIG. 6 is a cross sectional representation of a third embodiment of an electrode assembly 50c that includes a single electrode element E1. The embodiment of FIG. 6 is similar to the embodiment of FIG. 3A except the FIG. 6 embodiment does not include the layer of dielectric material. In the FIG. 6 embodiment, the electrode assembly 50c includes a sheet of anisotropic material 70 having a front face (facing towards the subject's skin in FIG. 6) and a rear face. This sheet 70 is similar to the sheet 70 described above in connection with FIG. 3A. In some embodiments, the sheet of anisotropic material 70 is a sheet of synthetic graphite. In some embodiments, the sheet of anisotropic material 70 is a sheet of pyrolytic graphite. In other embodiments, the sheet of anisotropic material 70 is graphite foil made from compressed high purity exfoliated mineral graphite (e.g., MinGraph^{®} 2010A Flexible Graphite). In other embodiments, the sheet of anisotropic material 70 is a sheet of graphitized polymer film. In other embodiments, the sheet of anisotropic material 70 is a sheet of pyrolytic carbon. In other embodiments, the anisotropic material may be boron nitride. In other embodiments, the sheet of anisotropic material 70 is a sheet of another conductive anisotropic material. In some embodiments (e.g., when the sheet of anisotropic material is a sheet of a synthetic graphite, such as pyrolytic graphite or the compressed high purity exfoliated mineral graphite or graphitized polymer film), the sheet of anisotropic material 70 is nonmetallic.

The electrode assembly 50c further includes at least one layer of biocompatible conductive material 60 disposed on the front face of the sheet 70, and the at least one layer of conductive material 60 has a biocompatible front surface. Note that the embodiment illustrated in FIG. 6, there is only a single layer of conductive material 60, and that single layer is biocompatible. But in alternative embodiments (not shown) there could be more than one layer, in which case only the front layer must be biocompatible. The at least one layer of conductive material 60 is configured to ensure good electrical contact between the device and the body. In a preferred embodiment, the at least one layer of conductive material 60 should cover the entire front face of the sheet of anisotropic material 70. The at least one layer of conductive material 60 may be the same size or larger (i.e., cover the same area or larger) than the sheet of anisotropic material 70. In some embodiments, the at least one layer of conductive material 60 comprises a single layer of hydrogel. In these embodiments, the hydrogel may have a thickness between 50 and 2000 µm, such as, from 100 to 1000 µm, or even 300 to 500 µm. In some embodiments, the at least one layer of conductive material 60 is a single layer of non-hydrogel biocompatible conductive adhesive as discussed above. In some embodiments, the at least one layer of conductive material 60 is a single layer of non-hydrogel biocompatible conductive adhesive such as the OMNI-WAVE products from FLEXcon, or the ARcare^{®} products from Adhesives Research, Inc., discussed above. Non-hydrogel conductive adhesives may comprise a waterless polymer with adhesive properties (for example, an acrylic polymer or a silicone polymer, or combination thereof) and a conductive filler. The conductive filler in the at least one layer of conductive material 60 should be non-metallic. In these embodiments, the biocompatible conductive adhesive may have a thickness between 10 and 2,000 µm, such as, from 20 to 1000 µm, or even 30 to 400 µm.

The electrode assembly 50c further includes a first electrode element E1 positioned behind the sheet 70. The first electrode element E1 includes a piece of metal 500 having a front face disposed in electrical contact with the rear face of the sheet 70. In the FIG. 6 embodiment, the front face of the piece of metal 500 is the first front face of the first electrode element E1. Accordingly, the FIG. 6 embodiment differs from the FIG. 3A or FIG. 5 embodiments by lacking a layer of dielectric material. The positional relationship between the first electrode element E1 and the sheet 70 in this FIG. 6 embodiment may be as described above in connection with FIG. 3A.

The electrode assembly 50c further includes a first rear layer of conductive material 80 positioned between the first front face of the first electrode element E1 (i.e., the front face of the piece of metal 500) and the rear face of the sheet 70. The first rear layer of conductive material 80 facilitates the electrical contact between the first front face of the first electrode element E1 and the rear face of the sheet 70. In the illustrated embodiment, the rear layer of conductive material 80 is a layer of hydrogel. But in alternative embodiments, a different conductive material (e.g., conductive grease, conductive adhesive including the non-hydrogel conductive adhesive described above, conductive tape, conductive composite, etc.) could be used.

The piece of metal 500 of the electrode element E1 is wired (e.g., using wires, traces on a flex circuit, etc.) to a lead 90, which supplies an AC voltage from an AC voltage generator (not shown) to the electrode element to generate the TTFields when the electrode assembly 50c is affixed to the subject's body for treatment.

The electrode assembly 50c may optionally include one or more additional electrode elements (not shown) having a structure identical to electrode element E1 and positioned to have the same functionality. In such case, the pieces of metal 500 of all the electrode elements may be wired together (e.g., using wires, traces on a flex circuit, etc.) to the lead 90.

In some embodiments that include only a single electrode element E1, the area of the sheet 70 is larger (e.g., at least 2 times larger, at least 4 times larger, or at least 10 times larger) than the area of the electrode element E1. In some embodiments that include a plurality of electrode elements (not shown) the area of the sheet 70 is larger (e.g., at least 2, 4, or 10 times larger) than the collective area of all of the electrode elements. When an AC voltage is applied to the electrode elements, the heat spreads out across the entire sheet 70, which minimizes or eliminates hot spots.

Similar to the FIG. 3A embodiment, the sheet of anisotropic material 70 in the FIG. 6 embodiment spreads out the heat and the current as described above in connection with the FIG. 3A embodiment, which eliminates or at least minimizes hot spots. This means that for a given applied AC voltage, the hottest point beneath the electrode assembly in the FIG. 6 embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 6 embodiment. And this increase in current will advantageously increase the efficacy of the TTFields treatment.

FIG. 7 is a cross sectional representation of a fourth embodiment of an electrode assembly 50d that includes a single electrode element E1. The FIG. 7 embodiment is similar to the FIG. 6 embodiment except that the first front face of the first electrode element E1 (i.e., the front face of the piece of metal 600) is positioned in direct contact with the rear face of the sheet 70 (instead of being electrically connected via an intervening layer of conductive material).

Similar to the FIG. 6 embodiment, the sheet of anisotropic material 70 in the FIG. 7 embodiment spreads out the heat and the current as described above in connection with the FIG. 3A embodiment, which eliminates or at least minimizes hot spots. This means that for a given applied AC voltage, the hottest point beneath the electrode assembly in the FIG. 7 embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 7 embodiment. And this increase in current will advantageously increase the efficacy of the TTFields treatment.

FIG. 8 is a cross sectional representation of a fifth embodiment of an electrode assembly 50e that includes a single electrode element E1. The FIG. 8 embodiment is similar to the FIG. 7 embodiment, but it adds a capacitor 700 connected in series with and behind the piece of metal 600. A similar addition of a capacitor 700 connected in series with and behind the piece of metal 600 could also be envisioned for the FIG. 6 embodiment.

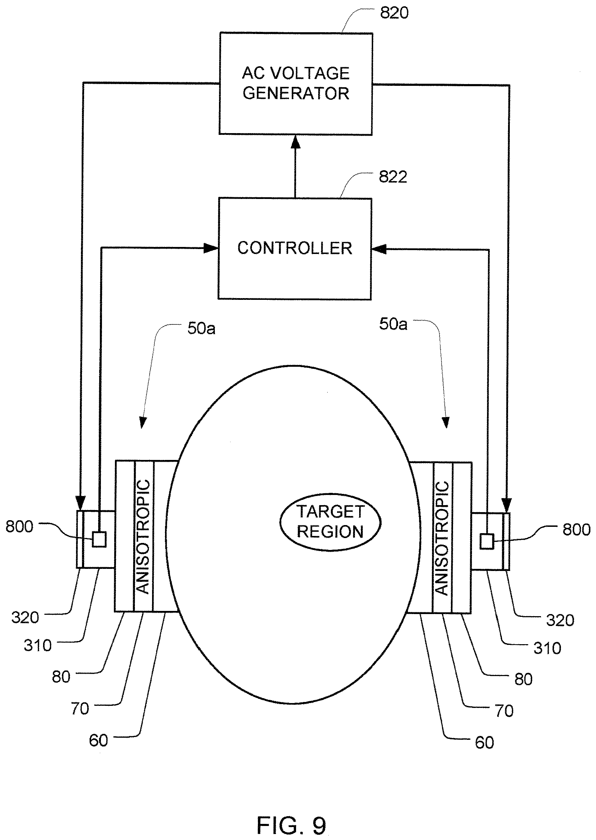

FIG. 9 shows how a pair of the FIG. 3A electrode assemblies 50a may be used to apply an alternating electric field to a target region in the subject's body. The subject could be a human or another mammal, including but not limited to rats and mice. (Note that any of the electrode assemblies described above in connection with FIGS. 5-8 may be used instead of the FIG. 3A electrode assemblies 50a shown here).

The method includes positioning a first electrode assembly 50a at a first position on or in the subject's body. (In the example depicted in FIG. 9, the first electrode assembly 50a is positioned on the subject's skin at the right of the subject's head facing a target region, e.g., a tumor.) The first electrode assembly 50a may be constructed as described earlier herein. In the FIG. 9 embodiment, the first electrode assembly 50a includes a first sheet 70 of anisotropic material 70 having a first front face and a first rear face. The first sheet 70 has a first thermal conductivity in a direction that is perpendicular to the first front face. Thermal conductivity of the first sheet 70 in directions that are parallel to the first front face of the sheet 70 is more than two times higher than the first thermal conductivity. The first sheet 70 has a first resistance in a direction that is perpendicular to the front face, and the resistance of the first sheet in directions that are parallel to the front face is less than half of the first resistance. During use, the first electrode assembly 50a is positioned so that the first front face of the first sheet 70 faces the target region.

The method also includes positioning a second electrode assembly 50a at a second position in or on the subject's body. (In the example depicted in FIG. 9, the second electrode assembly 50a is positioned on the subject's skin at the left of the subject's head facing the target region.) The second electrode assembly 50a may be constructed as described earlier herein. In the FIG. 9 embodiment, the second electrode assembly 50a includes a second sheet 70 of anisotropic material 70 having a second front face and a second rear face. The second sheet 70 has a second thermal conductivity in a direction that is perpendicular to the second front face. Thermal conductivity of the second sheet 70 in directions that are parallel to the second front face of the sheet 70 is more than two times higher than the second thermal conductivity. The second sheet 70 has a second resistance in a direction that is perpendicular to the front face, and the resistance of the second sheet in directions that are parallel to the front face is less than half of the second resistance. During use, the second electrode assembly 50a is positioned so that the second front face of the second sheet 70 faces the target region.

The method further includes applying an alternating voltage between the first electrode assembly 50a and the second electrode assembly 50a. The applying is performed after positioning the first electrode assembly 50a and the second electrode assembly 50a. The applying may be implemented by applying the alternating voltage between (i) a first electrode element disposed in electrical contact with the first rear face of the first sheet 70 and (ii) a second electrode element disposed in electrical contact with the second rear face of the second sheet 70.

In some embodiments, the first electrode assembly 50a further includes a first layer of biocompatible conductive material 60 disposed on the first front face of the first sheet 70. Correspondingly, the second electrode assembly further includes a second layer of biocompatible conductive material 60 disposed on the second front face of the second sheet 70. As described above, the biocompatible conductive material 60 may be hydrogel or may be a conductive grease, conductive adhesive including the non-hydrogel conductive adhesives discussed above, conductive tape, conductive composite, etc.

In some embodiments, the first electrode assembly 50a further includes a first rear layer of conductive material 80 (as described above) positioned between the first front face of the first electrode element of the first electrode assembly 50a and the first rear face of the first sheet 70. Correspondingly, the second electrode assembly further includes a second rear layer of conductive material 80 (as described above) positioned between the second front face of the second electrode element of the second electrode assembly and the second rear face of the second sheet 70.

In some embodiments, each of the first and second sheets of anisotropic material 70 is a sheet of synthetic graphite. In some embodiments, the sheet of anisotropic material 70 is a sheet of pyrolytic graphite. In other embodiments, each of the first and second sheets of anisotropic material 70 is graphite foil made from compressed high purity exfoliated mineral graphite (e.g., MinGraph^{®} 2010A Flexible Graphite, available from Mineral Seal Corp., Tucson Arizona). In other embodiments, the sheet of anisotropic material 70 is a sheet of graphitized polymer film. In other embodiments, the anisotropic material may be pyrolytic carbon. In other embodiments, the anisotropic material may be boron nitride. Other embodiments may utilize sheets of other conducting materials with anisotropic properties. In some embodiments (e.g., when the sheets of anisotropic material are sheets of synthetic graphite, such as pyrolytic graphite or compressed high purity exfoliated mineral graphite or graphitized polymer film), the sheets of anisotropic material 70 are nonmetallic.

The alternating voltage between the first electrode assembly and the second electrode assembly may be applied by an AC voltage generator 820. In some embodiments, the frequency of the alternating voltage is between 50 kHz and 1 MHz, or between 100 kHz and 500 kHz. In the illustrated example, the AC voltage generator is controlled by a controller 822. The controller 822 may use temperature measurements to control the amplitude of the current to be delivered via the first and second electrode assemblies 50a in order to maintain temperatures below a safety threshold (e.g., 41° C). This may be accomplished, for example, by measuring a first temperature of the first electrode element, measuring a second temperature of the second electrode element, and controlling the applying of the alternating voltage based on the first temperature and the second temperature, as described below.

FIG. 9 depicts one example of hardware that is suitable for this purpose. More specifically, temperature sensors 800 (e.g., thermistors) are positioned in thermal contact with respective electrode elements (for example, dielectric material 310 / layer of metal 320) within each of the electrode assemblies 50a. The temperature sensors 800 measure respective first and second temperatures (e.g., at first and second electrode elements in the first electrode assembly and second electrode assembly, respectively), and the controller 822 controls the output of the AC voltage generator 820 based on these temperatures.

Similar embodiments and methods are envisaged utilizing any of the electrode assemblies 50a-e, or combinations thereof, in place of either or both of the first electrode assembly 50a and the second electrode assembly 50a.

The electrode assemblies described herein may be used to apply an alternating electric field to a target region of a subject's body to treat a condition present in the subject. Such methods include positioning a first electrode assembly and a second electrode assembly on opposite sides of a target region of a subject's body, wherein the first and second electrode assemblies are as described herein, and applying an alternating electric field between the first and second electrode assemblies to treat a condition in the subject. The target region may be any region of the subject's body which is impaired by a condition or presents a condition. Suitable target regions include, but are not limited to, the brain, torso, abdomen, lung, breast, ovary, prostate, pancreas, stomach, intestine, colon, rectum, liver, or kidney of the subject. In some embodiments the target region may include a tumor or a cancer (one or more cancer cells).

By "treat" is meant to administer or apply a therapeutic, such as alternating electric fields, to a subject, such as a human or other mammal (for example, an animal model), that has a tumor (cancer) or has an increased susceptibility for developing cancer, in order to prevent or delay a worsening of the effects of the tumor (cancer), or to partially or fully reverse the effects of the tumor (cancer).

The alternating electric field used in the methods and/or embodiments described herein may have a frequency between 50 kHz to 2 MHz, 50 kHz and 1 MHz, 100 kHz and 1 MHz, 100 kHz and 500 kHz, 100 kHz and 300 kHz, or 150 kHz and 400 kHz. In some embodiments, the alternating electric field has a frequency of about 100 kHz, about 150 kHz, about 175 kHz, about 200 kHz, about 225 kHz, about 250 kHz, about 275 kHz, about 300 kHz, about 325 kHz, about 350 kHz, about 375 kHz, about 400 kHz, about 425 kHz, about 450 kHz, about 475 kHz, about 500 kHz, about 550 kHz, about 600 kHz, about 650 kHz, about 700 kHz, about 750 kHz, about 800 kHz, about 850 kHz, about 900 kHz, about 950 kHz, or about 1 MHz. The frequencies and frequency ranges listed above may be useful in any of the methods described herein.

In some methods and/or embodiments, the alternating electric field has an electric field strength between 0.1 V/cm and 20 V/cm, 0.1 V/cm and 10 V/cm, or 1 V/cm and 4 V/cm. The electric field strength ranges listed above may be useful in any of the methods and/or embodiments described herein.

Some methods and/or embodiments include positioning a first electrode assembly and a second electrode assembly on opposite sides of a target site in a subject's body, wherein the first and second electrode assemblies are as described herein, and applying an alternating electric field between the first and second electrode assemblies to treat a tumor or a cancer (region containing cancer cells) in the subject.

The methods and embodiments described herein may be used to treat a tumor or cancer in a subject. Such tumors (cancer) include, but are not limited to, brain cancers such as glioblastoma multiforme, lung cancers (e.g., malignant pleural mesothelioma, non-small cell lung cancer), brain metastases (e.g., from non-small cell lung cancer or melanoma), breast cancer, ovarian cancer, prostate cancer, pancreatic cancer, gastric cancer (e.g., gastroesophageal junction adenocarcinoma, gastric adenocarcinoma), intestinal cancer, colon cancer, rectal cancer, liver cancer, hepatocellular cancer, and uveal cancer (eye cancer).

In some embodiments, methods of treating a tumor or cancer in a subject include positioning a first electrode assembly and a second electrode assembly as described herein on opposite sides of a target region in a subject's body, and applying an alternating electric field between the first and second electrode assemblies, and further include administering one or more therapeutic substances, such as an anti-cancer drug. Suitable anti-cancer drugs include, but are not limited to, temozolomide, pemetrexed, cisplatin, carboplatin, paclitaxel, nab-paclitaxel, doxorubicin, cyclophosphamide, trastuzumab, atezolizumab, gemcitabine, mebendazole, sorafenib, oxaliplatin, capecitabin, fluorouracil, leucovorin, ABT-751, ABT-414 (depatuxizumab mafodotin) and bevicizumab.

In some embodiments, methods of treating a tumor or cancer in a subject include positioning a first electrode assembly and a second electrode assembly as described herein on opposite sides of a target region in a subject's body, and applying an alternating electric field between the first and second electrode assemblies, and further include administering one or more checkpoint inhibitors. Suitable checkpoint inhibitors include, but are not limited to, PD-1 inhibitors, PDL-1 inhibitors, and CTLA-4 inhibitors. For example, one or more of pembrolizumab, nivolumab, cemiplimab, atezolimumab, durvalumab, avelumab, and ipilumab may be administered.

In some embodiments, methods of treating a tumor or cancer in a subject include positioning a first electrode assembly and a second electrode assembly as described herein on opposite sides of a target region in a subject's body, and applying an alternating electric field between the first and second electrode assemblies, and further include administering radiation therapy. Radiation therapy can include, but is not limited to, photon radiation (e.g., x-ray therapy, gamma ray therapy) and particle radiation (e.g., electron therapy, proton therapy, neutron therapy, carbon ion therapy, alpha particle therapy, beta particle therapy). Radiation therapy can be administered, for example, externally or internally (e.g., brachytherapy).

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering one or more agents that impede or prevent a DNA repair mechanism. For example, agents that impede or prevent a DNA repair mechanism include E2F inhibitors, CDK inhibitors, and PARP inhibitors. Suitable E2F inhibitors include, but are not limited to, HLM006474, MRT00033659, YKL-5-124-TFA, and YKL-5-124. Suitable CDK inhibitors include, but are not limited to, abemaciclib, ribociclib, trilaciclib, ibrance, lerociclib, alvocidib, roniciclib, riviciclib, milciclib, RGB-286638, NSN3106729, PHA-793887, R547, indirubin, NU6102, bohemine, CDK9-IN-7, CGP60474, purvalanol A, PF-06873600, nimbolide, FN-1501, AG-024322, ON123300, G1T28, G1T38, AMG925, SHR-6390, BPI-1178, BPI-16350, FCN437, birociclib, BEBT-209, Ty-302, TQB-3616, HS-10342, PF-06842874, CS-2002, MM-D37K, CDK4/6-IN-2, SU9516, and AT7519. Suitable PARP inhibitors include, but are not limited to, niraparib, olaparib, and rucaparib. In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering an E2F inhibitor. In some embodiments, methods of treating a tumor or cancer in a subject include positioning a first electrode assembly and a second electrode assembly as described herein on opposite sides of a target region in a subject's body, and applying an alternating electric field between the first and second electrode assemblies, and further include administering a CDK 4/6 inhibitor. In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering a PARP inhibitor.

In some embodiments, methods of treating a tumor or cancer in a subject include positioning a first electrode assembly and a second electrode assembly as described herein on opposite sides of a target region in a subject's body, and applying an alternating electric field between the first and second electrode assemblies, and further include administering radiation therapy and administering one or more agents that impede or prevent a DNA repair mechanism, as described herein.

In some embodiments, methods of treating a tumor or cancer in a subject include positioning a first electrode assembly and a second electrode assembly as described herein on opposite sides of a target region in a subject's body, and applying an alternating electric field between the first and second electrode assemblies, and further include administering an aurora kinase inhibitor. Suitable aurora kinase inhibitors include, but are not limited to AZD1152, danusertib (PHA-739358), AT9283, PF-03814735, and AMG 900.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering an agent that increases the sensitivity of the tumor or cancer (or a cancer cell of which the tumor is comprised; or a cancer cell or cancer cell cluster not associated with a tumor) to treatment with the alternating electric field. In some embodiments, the sensitivity of the tumor or cancer cell to alternating electric fields can be increased by altering the cyclin D1 pathway. In some embodiments, the cyclin D1 pathway can be altered by administration of one or more of mammalian target of rapamycin (mTOR) inhibitor, an Akt inhibitor, a phosphatidylinositol 3-kinase (PI3K) inhibitor, an Src inhibitor, a focal adhesion kinase (FAK) inhibitor, or a glycogen synthase kinase 3β (GSK3β) inhibitor.

Accordingly, methods of treating a tumor or cancer in a subject include positioning a first electrode assembly and a second electrode assembly as described herein on opposite sides of a target region in the subject's body, and applying an alternating electric field between the first and second electrode assemblies, and further include administering an mTOR inhibitor. Suitable mTOR inhibitors include, but are not limited to, torkinibs, everolimus, temsirolimus, everolimus, CC-223, MKK-1, AZD8055, AZD02114, INK-128, 051-027, dactolisib, BGT226, SF1126, PKI-587, NVPBE235, sapanisertib, AZD2014, BEZ235, XL765, GDC0980, SF1126, PF-04691502, PF-05212384 (gedatolisib, PKI-587), LY3023414, XL795 (voxtasilib), Bimiralisib (PQR309), Paxalisib (GDC-0084), DS-7423, PKI-179, GSK458V, P7170, SB2343, PI-103, NU7441, KU-0063794, Ridaforolimus (deforolimus, MK-8669), Torin 1, Torin 2, OSI-027, GSK1059615, WYE-354, Vistusertib (AZD2014), WYE-125132, Palomid 529 (P529), WYE-687, XL388, MHY1485, LY3023414 (Samotolisib), GNE-447, CC-115, Zotarolimus (ABT-578), PQR620, SF2523, mTor inhibitor-1,2,3 or 8, PQR626, WAY-600, PF-04979064 , 3BDO, Dihydromyricetin, ETP-46464, PKI-402, Cyclovirbuxine D, CZ415, VS-5584, (+)-usunic acid, RMC-5552, PQR530, JR-AB2-011, Arnicolide D or TML-6.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering an Akt inhibitor. Suitable Akt inhibitors include, but are not limited to, any composition or compound that inhibits Akt, inhibits phosphorylation of Akt, inhibits phosphorylated Akt, or inhibits degradation of cyclin D1. In some methods and/or embodiments, an Akt inhibitor can be, but is not limited to, lapatinib, H8, H-89, NL-71-101, GSK690693, 7-azaindole, 6-phenylpurine derivatives, pyrrolo[2,3-d]pyrimidine derivatives, CCT128930, 3-aminopyrrolidine, anilinotriazole derivatives, spiroindoline derivatives, AZD5363, ipatasertib (GDC-0068, RG7440), A-674563, A-443654 ,AT7867, AT13148, Afuresertib (GSK2110183), 2 pyrimidyl-5-amidothiophene derivative (DC120), uprosertib (GSK2141795), 2,3-diphenylquinoxaline derivatives, triazolo[3,4-f][1,6]naphthyridin-3(2H)-one derivative (MK 2206), Edelfosine (1-O-octadecyl-2-O-methyl-rac-glycerol-3-phosphocholine, ET-18-OCH3) ilmofosine (BM 41.440), miltefosine (hexadecylphosphocholine, HePC), perifosine (D 21266), erucylphosphocholine (ErPC), erufosine (ErPC3, erucylphosphohomocholine), Indole-3-carbinol, 3-chloroacetylindole, diindolylmethane, diethyl-6-methoxy-5,7-dihydroindolo-[2,3 b]carbazole-2,10-dicarboxylate (SR13668), OSU-A9, PH-316, PHT-427, PIT-1, PIT-2, M-PIT-1, [(1-methyl-1H-pyrazol-4-yl)carbonyl]-N'-(3-bromophenyl)-thiourea, Triciribine (TCN, NSC-154020), triciribine mono-phosphate active analogue (TCN-P), 4-amino-pyrido[2,3-d]pyrimidine derivative, API-1, 3-phenyl-3H-imidazo[4,5-b]pyridine derivatives, ARQ-092, BAY-1125976, 3-methyl-xanthine, quinolone-4-carboxamide and 2-[4-(cyclohexa 1,3-dien-1-yl)-1H-pyrazol-3-yl]phenol, 3-oxo-tirucallic acid, 3α- and 3β-acetoxy tirucallic acids, acetoxy tirucallic acid, Lactoquinomycin, Frenolicin B, kalafungin, medermycin, Boc-Phe-vinyl ketone, 4 hydroxynonenal-(4-HNE), 1,6-naphthyridinone derivatives, imidazo-1,2-pyridine derivatives), Rigosertib (ON-01910), Triciribine, Honokiol, Miransertib (ARQ-092), Borussertib, SC66, A-674563, TIC10 analogue, Urolithin B, ABTL-0821, Loureirin A, Homosalate, Deguelin, Resibfogenin, Terameprocol, Oroxin B, LM22B-10, Amarogentin, Oridonin, Praeruptorin A, or Scutellarin.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering a PI3K inhibitor. Suitable PI3K inhibitors include, but are not limited to, GDC-0941, TG100-115, CH5132799, PX-866, XL147, ZSTK474, BKM-120, BAY80-6946, AZD8835, WX-037, AZD8186, KA2237, CAL-120, ME401, AMG319, GSK2636771, INCB050465, INK-1117, TGR-1202, RP6530, GDC-0032, BYL719, BGT226, IPI-145, CAL-101, AMG511, ADZ6482, MLN1117, 3-Hydroxyanthranilic acid, Hispidulin, Pectolinarin, or Cinobufagin.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering an Src inhibitor. Suitable Src inhibitors include, but are not limited to, Dasatinib (BMS-354825), Ponatinib (AP24534), Saracatinib (AZD0530), Bosutinib (SKI-606), Dehydroabietic acid (DAA, DHAA), PP2, Ginkgolic acid C17:1 (GAC 17:1), DGY-06-116, Doramapimod (BIRB 796), Apatinib, Pelitinib (EKB-569), Resveratrol, KX2-391 (Tirabanibulin), NVP-BHG712, ENMD-2076, PRT062607 (P505-15, BIIB057, PRT-2607), PP1, MNS(3,4-Methylenedioxy-β-nitrostyrene), Doramapimod (BIRB 796), WH-4-023, RK24466, KX1-004, 7-Hydroxychromone, AD-80. Repotrectinib (TPX-0005), Quercetin (NSC 9221, Sophoretin, C.I. 75720), SU 6656, Src Inhibitor 1 (CAS 179248-59-0), CCT196969, Myristic acid (Tetradecanoic acid), eCF506, 1-Naphthyl PP1 (1-NA-PP 1), AMG-47a. ON123300, UM-164, MLR-1023, PD173955, AZD0424, PD180970 or HG-7-85-01.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering a focal adhesion kinase (FAK) inhibitor. Suitable FAK inhibitors include, but are not limited to, Defectanib (VS-6063), Solanesol (nonaisoprenol), PF-00562271 Besylate (PF-562271), PF-562271 (PF-00562271), PRT062607 (P505-15, BIIB057, PRT-2607), PF-573228 TAE226 (NVP-TAE226), PF-562271 HCl, BI-4464, Y15, GSK2256098, PND-1186(VS-4718), PF-431396, FAK inhibitor 14 (cas 4506-66-5) or Rebastinib.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering a glycogen synthase kinase 3β (GSK3β) inhibitor. Suitable GSK3β inhibitors include, but are not limited to, Lithium, Zinc, Tungstate, Naproxen, Cromolyn, Famotidine, Olanzapine, Pyrimidine derivatives, CT98014, CT98023, CT99021, TWS119, Indirubine, 6-BIO, Hymenialdisine, Dibromocanthareline, Meridianin, Arylindolemaleimide, SB-216763, SB-41528, Thiazoles, AR-A014418, AZD-1080, Paullones, Kenpaullone, Alsterpaullone, Cazpaullone, Alosines, Manzamins, Manzamin A, Furanosesquiterpenes, Palinurine, Tricantine, L803-mts, Thiadiazolidindiones, TDZD-8, NP00111, NP031115, NP031112 (tideglusib), Halomethylketones (HMK-32), L803-mts, CH1R99021, CT99021, TWS119, Aloisines, 9-ING-41, 1-Azakenpaullone, IM-12, CHIR-98014, or LY2090314.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering a chloride intracellular channel (e.g., CLIC1 and/or CLIC4) inhibitor. Suitable CLIC inhibitors include, but are not limited to, indanyloxyacetic acid-94 (IAA94), niflumic acid, diphenylamine-2-carboxylic acid (DPC), 4,4'-Diisothiocyano-2,2'-stilbenedisulfonic Acid, Disodium Salt (DIDS), anthracene derivatives, stilbenes, and heavy metal ions, blockbenzimidazole, clofubric acid, benzofurans, propionic acid (CPP), 4-acetamido-4-isothiocyanatostilbene-2,2-disulfonic acid (SITS), 5-nitro-2-(3-phenylpropyl-amino) benzoic acid (NPPB), Chlorotoxin, mibefradil, Calix[4]arene, Clomiphene, Cd2+, Gd3+, glibenclaimide, flufenamic acid, inositol-tetrabisphosphate, mefloquinand or fluoxetine.

In some embodiments, methods of treating a tumor or cancer in a subject include positioning a first electrode assembly and a second electrode assembly as described herein on opposite sides of a target region in the subject's body, and applying an alternating electric field between the first and second electrode assemblies, and further include administering a fibroblast growth factor (FGF) inhibitor. In some methods and/or embodiments, the FGF inhibitor specifically inhibits or decreases FGF expression. In some methods and/or embodiments, the FGF inhibitor specifically binds to a nucleic acid that encodes an FGF. In some methods and/or embodiments, the FGF is FGF-21, FGF-19, FGF-7, FGF-basic. In some methods and/or embodiments, the FGF inhibitor can be a small molecule, peptide, protein, antibody, or nucleic acid (e.g. siRNA). Examples of FGF inhibitors include, but are not limited to, soluble fibroblast growth factor receptor (FGFR), such as soluble FGFR3, and soluble decoy receptors, such as FGF-Trap that is a soluble decoy receptor fusion protein that binds FGF-2.

In some embodiments, methods of treating a tumor or cancer in a subject include positioning a first electrode assembly and a second electrode assembly on opposite sides of a target region in the subject's body, and applying an alternating electric field between the first and second electrode assemblies, and further include administering a fibroblast growth factor receptor (FGFR) inhibitor. Suitable examples of FGFR inhibitors include, but are not limited to, BAY 1179470, FPA144, PRO-001, RG7444, SSR128129E, AZD4547, BAY1163877, BGJ398, CH5183284, Erdafitinib, LY2874455, Lucitanib/E3810, Nintedanib, Pazopanib, Roblitinib, PD-161570, CP-547632, Infigratinib (BGJ398), PD173074, SSR128129E, PD-166866, ASP5878, H3B-6527, NSC12, BO-264, Sulfatinib, Fisogatinib (BLU-554), FIIN-2, Futibatinib (TAS-120), BLU9931, Pemigatinib (INCB054828), Zoligratinib (Debio-1347), Alofanib (RPT835), PRN1371, Ferulic Acid, ODM-203, and Derazantinib (ARQ-087).

In some embodiments, methods of treating a tumor or cancer in a subject include positioning a first electrode assembly and a second electrode assembly on opposite sides of a target region in the subject's body, and applying an alternating electric field between the first and second electrode assemblies, and further include administering one or more of lenvatinib and bevacizumab.

In some embodiments, a method of treating non-small cell lung cancer includes positioning a first electrode assembly and a second electrode assembly on opposite sides of a subject's torso, and applying an alternating electric field between the first and second electrode assemblies, wherein the alternating electric field has a frequency of about 50-350 kHz, such as about 150 kHz. In some embodiments, a method of treating non-small cell lung cancer includes applying an alternating electric field to a subject's torso with two or more electrode assemblies as described herein, and further includes administering docetaxel. In some embodiments, a method of treating non-small cell lung cancer includes applying an alternating electric field to a subject's torso with two or more electrode assemblies as described herein, wherein the alternating electric field has a frequency of about 50-350 kHz, such as about 150 kHz, and further includes administering a checkpoint inhibitor. Suitable checkpoint inhibitors include, but are not limited to, pembrolizumab, nivolumab, cemiplimab, atezolimumab, durvalumab, avelumab, and ipilumab. In some embodiments, a method of treating non-small cell lung cancer includes applying an alternating electric field to a subject's torso with two or more electrode assemblies as described herein, wherein the alternating electric field has a frequency of about 50-350 kHz, such as about 150 kHz, and further includes administering docetaxel and pembrolizumab.

In some embodiments, a method of treating malignant pleural mesothelioma includes applying an alternating electric field to a subject's torso (e.g., lung) with two or more electrode assemblies as described herein, wherein the alternating electric field has a frequency of about 50-350 kHz, such as about 150 kHz. In some embodiments, a method of treating malignant pleural mesothelioma includes applying an alternating electric field to a subject's torso (e.g., lung) with two or more electrode assemblies as described herein, wherein the alternating electric field has a frequency of about 50-350 kHz, such as about 150 kHz, and further includes administering a chemotherapeutic agent. In some embodiments, a method of treating malignant pleural mesothelioma includes applying an alternating electric field to a subject's torso (e.g., lung) with two or more electrode assemblies as described herein, wherein the alternating electric field has a frequency of about 50-350 kHz, such as about 150 kHz, and further includes administering one or more of pemetrexed, cisplatin and carboplatin.

In some embodiments, a method of treating ovarian cancer includes positioning a first electrode assembly and a second electrode assembly on opposite sides of a subject's abdomen, and applying an alternating electric field between the first and second electrode assemblies, wherein the alternating electric field has a frequency of about 100-400 kHz, such as about 200 kHz. In some embodiments, a method of treating ovarian cancer includes applying an alternating electric field to a subject's abdomen with two or more electrode assemblies as described herein, wherein the alternating electric field has a frequency of about 100-400 kHz, such as about 200 kHz, and further includes administering paclitaxel.

In some embodiments, a method of treating glioblastoma multiforme (GBM) includes positioning a first electrode assembly and a second electrode assembly on opposite sides of a subject's head, and applying an alternating electric field between the first and second electrode assemblies wherein the alternating electric field has a frequency of about 100-400 kHz, such as about 200 kHz. In some embodiments, a method of treating glioblastoma multiforme includes applying an alternating electric field to a target region of a subject's brain with two or more electrode assemblies as described herein, wherein the alternating electric field has a frequency of about 100-400 kHz, such as about 200 kHz, and further includes administering one or more of temozolomide and a checkpoint inhibitor. Suitable checkpoint inhibitors include, but are not limited to, pembrolizumab, nivolumab, cemiplimab, atezolimumab, durvalumab, avelumab, and ipilumab.

In some embodiments, a method of treating glioblastoma multiforme includes applying an alternating electric field to a subject's head with two or more electrode assemblies as described herein, wherein the alternating electric field has a frequency of about 100-400 kHz, such as about 200 kHz, and further includes administering temozolomide. In some embodiments, a method of treating glioblastoma multiforme includes applying an alternating electric field to a subject's head with two or more electrode assemblies as described herein, wherein the alternating electric field has a frequency of about 100-400 kHz, such as about 200 kHz, and further includes administering pembrolizumab. In some embodiments, a method of treating glioblastoma multiforme includes applying an alternating electric field to a subject's head with two or more electrode assemblies as described herein, wherein the alternating electric field has a frequency of about 100-400 kHz, such as about 200 kHz, and further includes administering temozolomide and administering pembrolizumab.

In some embodiments, a method of treating glioblastoma multiforme includes applying an alternating electric field to a subject's head with two or more electrode assemblies as described herein, wherein the alternating electric field has a frequency of about 100-400 kHz, such as about 200 kHz, and further includes administering radiation therapy (e.g., 60 Gy given in 30 2 Gy fractions). In some embodiments, a method of treating glioblastoma multiforme includes applying an alternating electric field to a subject's head with two or more electrode assemblies as described herein, wherein the alternating electric field has a frequency of about 100-400 kHz, such as about 200 kHz, and further includes administering radiation therapy (e.g., 60 Gy given in 30 2 Gy fractions) and administering temozolomide.

In some embodiments, a method of treating glioblastoma multiforme includes applying an alternating electric field to a subject's head with two or more electrode assemblies as described herein, wherein the alternating electric field has a frequency of about 100-400 kHz, such as about 200 kHz, and further includes administering bevacizumab. In some embodiments, a method of treating glioblastoma multiforme includes applying an alternating electric field to a subject's head with two or more electrode assemblies as described herein, wherein the alternating electric field has a frequency of about 100-400 kHz, such as about 200 kHz, and further includes administering niraparib.

In some embodiments, a method of treating gastric cancer includes applying an alternating electric field to a subject's abdomen with two or more electrode assemblies as described herein, wherein the alternating electric field has a frequency of about 50-350 kHz, such as about 150 kHz. In some embodiments, a method of treating gastric cancer includes applying an alternating electric field to a subject's abdomen with two or more electrode assemblies as described herein, wherein the alternating electric field has a frequency of about 50-350 kHz, such as about 150 kHz, and further includes administering trastuzumab.

In some embodiments, a method of treating gastric cancer includes applying an alternating electric field to a subject's abdomen with two or more electrode assemblies as described herein, wherein the alternating electric field has a frequency of about 50-350 kHz, such as about 150 kHz, and further includes administering one or more chemotherapeutic agents, for example oxaliplatin and capecitabine. In some embodiments, a method of treating gastric cancer includes applying an alternating electric field to a subject's abdomen with two or more electrode assemblies as described herein, wherein the alternating electric field has a frequency of about 50-350 kHz, such as about 150 kHz, and further includes administering one or more of oxaliplatin, capecitabine, and trastuzumab.

In some embodiments, a method of treating pancreatic cancer includes applying an alternating electric field to a subject's abdomen (e.g., pancreas) with two or more electrode assemblies as described herein, wherein the alternating electric field has a frequency of about 50-350 kHz, such as about 150 kHz. In some embodiments, a method of treating pancreatic cancer includes applying an alternating electric field to a subject's abdomen (e.g., pancreas) with two or more electrode assemblies as described herein, wherein the alternating electric field has a frequency of about 50-350 kHz, such as about 150 kHz, and further includes administering one or more of gemcitabine and nab-paclitaxel.

In some embodiments, a method of treating hepatocellular carcinoma includes applying an alternating electric field to a subject's torso (e.g., liver) with two or more electrode assemblies as described herein, wherein the alternating electric field has a frequency of about 50-350 kHz, such as about 150 kHz. In some embodiments, a method of treating hepatocellular carcinoma includes applying an alternating electric field to a subject's torso (e.g., liver) with two or more electrode assemblies as described herein, wherein the alternating electric field has a frequency of about 50-350 kHz, such as about 150 kHz, and further includes administering sorafenib.

In some embodiments, a method of treating metastatic uveal melanoma includes applying an alternating electric field to a subject's head (e.g., eye) with two or more electrode assemblies as described herein, wherein the alternating electric field has a frequency of between about 100 kHz to about 500 kHz (e.g., about 150 kHz, about 200 kHz, or about 250 kHz). In some embodiments, a method of treating metastatic uveal melanoma includes applying an alternating electric field to a subject's head (e.g., eye) with two or more electrode assemblies as described herein, wherein the alternating electric field has a frequency of between about 100 kHz to about 500 kHz (e.g., about 150 kHz, about 200 kHz, or about 250 kHz) and further includes administering a checkpoint inhibitor. Suitable checkpoint inhibitors include, but are not limited to, pembrolizumab, nivolumab, cemiplimab, atezolimumab, durvalumab, avelumab, and ipilumab. In some embodiments, a method of treating metastatic uveal melanoma includes applying an alternating electric field to a subject's head (e.g., eye) with two or more electrode assemblies as described herein, wherein the alternating electric field has a frequency of between about 100 kHz to about 500 kHz (e.g., about 150 kHz, about 200 kHz, or about 250 kHz) and further includes administering one or more of nivolumab and ipilimumab.

Some methods and/or embodiments include applying an alternating electric field to a target region of a subject's body to increase permeability of intercellular tight junctions between epithelial cells. For example, some methods include positioning a first electrode assembly and a second electrode assembly as described herein on opposite sides of a subject's head, and applying an alternating electric field between the first and second electrode assemblies to increase permeability of the intercellular tight junctions (TJs) between brain capillary endothelial cells (i.e., the blood brain barrier). In other embodiments, the methods include positioning a first electrode assembly and a second electrode assembly on opposite sides of a subject's abdomen, and applying an alternating electric field between the first and second electrode assemblies to increase permeability of the intestinal epithelium. Such increased permeability of intercellular tight junctions between epithelial cells may be useful in increasing absorption of substances, such as pharmaceuticals. In some embodiments, such methods further comprise administering a therapeutic substance to the subject. In some embodiments the alternating electric field that is useful for increasing the permeability of intercellular tight junctions has a frequency between about 50 kHz and about 500 kHz, about 100 kHz and about 300 kHz, or about 100 kHz and about 190 kHz. In some embodiments the frequency is about 150 kHz, about 160 kHz, about 170 kHz, about 180 kHz, or about 190 kHz.

Some methods and/or embodiments include positioning a first electrode assembly and a second electrode assembly as described herein on opposite sides of a target region in a subject's body, and applying an alternating electric field between the first and second electrode assemblies to increase permeability of a cell membrane, in particular of a cancer cell membrane. Such cancer cell membrane can be the membrane of any cancer cell, for example, brain cancer cell such as glioblastoma multiforme, lung cancer cell (e.g., malignant pleural mesothelioma, non-small cell lung cancer), brain metastases cell (e.g., from non-small cell lung cancer or melanoma), breast cancer, ovarian cancer cell, prostate cancer cell, pancreatic cancer cell, gastric cancer cell (e.g., gastroesophageal junction adenocarcinoma, gastric adenocarcinoma), intestinal cancer cell, colon cancer cell, rectal cancer cell, liver cancer, hepatocellular cancer cell, and uveal cancer cell. In some embodiments, such methods further comprise administering a therapeutic substance to the subject or in the vicinity of the cancer cell. In some embodiments the alternating electric field that is useful for increasing the permeability of cell membrane has a frequency between about 50 kHz and about 1 MHz, between about 50 kHz and about 500 kHz, or between about 100 kHz and about 300 kHz.

Some methods and/or embodiments include positioning a first electrode assembly and a second electrode assembly as described herein on opposite sides of a subject's head, and applying an alternating electric field between the first and second electrode assemblies to treat or reduce the symptoms of a neurodegenerative disease. For example, such neurodegenerative disease could be, but is not limited to, one or more of amyotrophic lateral sclerosis (ALS), Parkinson's disease, Alzheimer's disease, Huntington's disease, multiple sclerosis (MS), neurofibromatosis, or dementia. In some embodiments, a method of treating a neurodegenerative disease includes applying an alternating electric field to a subject's brain to inhibit GSK3β.

Some methods and/or embodiments include positioning a first electrode assembly and a second electrode assembly as described herein on opposite sides of a subject's body, and applying an alternating electric field between the first and second electrode assemblies to treat or reduce the symptoms of a neurodegenerative disorder, and further include administering a therapeutic agent for treatment of the neurodegenerative disorder. Therapeutic agents for treatment of neurodegenerative disorders include, but are not limited to, GSK3β inhibitor, a cholinesterase inhibitor, an anti-amyloid monoclonal antibody (e.g., aducanumab), a dopamine agonist, a monoamine oxidase B (MAO-B) inhibitor, a catechol O-methyltransferase inhibitor, or an anticholinergic. In some embodiments the therapeutic agent is one or more of 9-ING-41, HY-130795, TWS119, Tideglusib, SAR502250, AR-A014418, TDZD8, Kenpaullone, Cromoyb sodium, SB415286, IM-12, CP21R7, GNF4877, 1-Azakenpaullone, or Indirubin-3'-monoxime, tacrine, galantamine, liquiritigenin, memantine, rivastigmine, donepezil, ciprofloxacin, celecoxib, tauroursodeoxycholic acid, sodium phenylbutyrate, levodopa, carbidopa, pramipexole, ropinirole, rotigotine, apomorphine, selegiline, rasagiline, safinamide, entacapone, opicapone, tolcapone, benztropine, trihexyphenidyl, amantadine, riluzole, edaravone, and 1-3,4-dihydroxyphenylalanine.

Some methods and/or embodiments include positioning a first electrode assembly and a second electrode assembly as described herein on opposite sides of a target region, and applying an alternating electric field between the first and second electrode assemblies to treat or reduce the symptoms of an autoimmune disease, wherein the target region includes tissue that is being attacked by the autoimmune disease. For example, in Rasmussen encephalitis (RE), the immune system attacks a single hemisphere of a person's brain, accordingly in methods for treating RE the target region may be the subject's head or brain. In lupus nephritis the immune system attacks a person's kidneys. The target region for lupus nephritis is to place one pair of electrodes on the subject's body in front of and behind the kidneys and/or associated draining lymph nodes, and, optionally, a second pair of electrodes on the sides of the subject's body at a height that corresponds to the kidneys and/or the associated draining lymph nodes. Methods of treating type 1 diabetes may include a target region of the subject's pancreas. Methods of treating rheumatoid arthritis may include a target region of the relevant joints and/or associated draining lymph nodes. Methods of treating polymyositis may include a target region of the subject's relevant muscles and/or associated draining lymph nodes.

Some methods and/or embodiments include positioning a first electrode assembly and a second electrode assembly as described herein on opposite sides of a target region of a subject, and applying an alternating electric field between the first and second electrode assemblies to treat a subject infected with a virus that relies on electrostatic interaction with a receptor, wherein the target region comprises one or more cells infected with the virus. For example, the virus can be a coronavirus or lentivirus. In some embodiments the alternating electric field has a frequency between 50 kHz and 1 MHz, or between 100 kHz and 500 kHz. In some embodiments the alternating electric field has a frequency of about 150 kHz.

Some methods and/or embodiments include positioning a first electrode assembly and a second electrode assembly as described herein on opposite sides of a target region of a subject, and applying an alternating electric field between the first and second electrode assemblies to treat a subject infected with a lentivirus or coronavirus and further includes administering an antiviral therapeutic to the subject. Suitable examples of antiviral therapeutics include, but are not limited to, remdesivir (Veklury), Avigan (favilavir), Olumiant and Baricinix (baricitinib), hydroxychloroquine/chloroquine, Casirivimab and imdevimab (formerly REGN-COV2), PTC299, Leronlimab (PRO 140), Bamlanivimab (LY-CoV555), Lenzilumab, Ivermectin, RLF-100 (aviptadil), Metformin (Glucophage, Glumetza, Riomet), AT-527, Actemra (tocilizumab), Niclocide (niclosamide), Convalescent plasma, Pepcid (famotidine), Kaletra (lopinavir-ritonavir), Remicade (infliximab), AZD7442, AZD7442, CT-P59, Heparin (UF and LMW), VIR-7831 (GSK4182136), JS016, Kevzara (sarilumab), SACCOVID (CD24Fc), Humira (adalimumab), COVI-GUARD (STI-1499), Dexamethasone (Dextenza, Ozurdex, others), PB1046, Galidesivir, Bucillamine, PF-00835321 (PF-07304814), Eliquis (Apixaban), Takhzyro (lanadelumab), Hydrocortisone, Ilaris (canakinumab), Colchicine (Mitigare, Colcrys), BLD-2660, Avigan (favilavir/avifavir), Rhu-pGSN (gelsolin), MK-4482, TXA127, LAM-002A (apilimod dimesylate), DNL758 (SAR443122), INOpulse, ABX464, AdMSCs, Losmapimod, Mavrilimumab, and Calquence (acalabrutinib).

Some methods and/or embodiments include positioning a first electrode assembly and a second electrode assembly as described herein on opposite sides of a subject's torso (e.g., lungs), and applying an alternating electric field between the first and second electrode assemblies to treat or reduce the symptoms of acute respiratory distress syndrome. In some embodiments the acute respiratory distress syndrome may have been caused by a viral infection, such as a coronavirus infection.

While the present invention has been disclosed with reference to certain embodiments, numerous modifications, alterations, and changes to the described embodiments are possible without departing from the sphere and scope of the present invention, as defined in the appended claims. Accordingly, it is intended that the present invention not be limited to the described embodiments, but that it has the full scope defined by the language of the following claims, and equivalents thereof.

## Claims

1. An apparatus for applying an alternating electric field to a subject's body, the apparatus comprising:
a sheet of anisotropic material (70) having a front face and a rear face, the front face being configured to face the subject's skin, wherein the sheet of anisotropic material (70) has a first resistance in a direction that is perpendicular to the front face thereof, and a resistance of the sheet of anisotropic material (70) in directions that are parallel to the front face thereof is less than half of the first resistance;
at least one layer of conductive material (60) disposed in electrical contact with the front face of the sheet of anisotropic material (70), wherein the at least one layer of conductive material (60) has a biocompatible front surface; and
at least one electrode element (E1, E2) positioned behind the sheet of anisotropic material (70), wherein the at least one electrode element (E1, E2) has a front face disposed in electrical contact with the rear face of the sheet of anisotropic material (70).

2. The apparatus of claim 1, comprising a plurality of electrode elements (E1, E2), wherein each of the electrode elements (E1, E2) comprises a layer of dielectric material (310) having a front face and a rear face, the front face of the layer of dielectric material (310) being the front face of the electrode element (E1, E2), and a layer of metal (320) disposed on the rear face of the layer of dielectric material (310), and further comprising:
a rear layer of conductive material (80) positioned between the front faces of the electrode elements (E1, E2) and the rear face of the sheet of anisotropic material (70).

3. The apparatus of claim 1, wherein each of the at least one electrode element (E1, E2) comprises a layer of dielectric material (310) having a front face and a rear face, the front face of the layer of dielectric material (310) being the front face of the at least one electrode element (E1, E2), a layer of metal (320) disposed on the rear face of the layer of dielectric material (310), and a rear layer of conductive material (380) positioned between the front face of each of the at least one electrode element (E1, E2) and the rear face of the sheet of anisotropic material (70).

4. The apparatus of claim 2 or 3, wherein the rear layer of conductive material (80; 380) comprises a conductive adhesive, optionally the conductive adhesive comprises an adhesive polymer and carbon powder, particles, fibers, flakes or nanotubes.

5. The apparatus of claim 1, wherein the at least one electrode element (E1, E2) comprises a piece of metal (500; 600) having a front face, the front face of the piece of metal (500; 600) being the front face of the at least one electrode element (E1, E2).

6. The apparatus of claim 5, further comprising:
a rear layer of conductive material (80; 380) positioned between the front face of the at least one electrode element (E1, E2) and the rear face of the sheet of anisotropic material (70).

7. The apparatus of claim 5, wherein the front face of the at least one electrode element (E1, E2) is positioned in direct contact with the rear face of the sheet of anisotropic material (70).

8. The apparatus of claim 1, wherein the sheet of anisotropic material (70) is a sheet of graphite.

9. The apparatus of claim 1, wherein the sheet of anisotropic material (70) is a sheet of pyrolytic graphite, a graphite foil of compressed high-purity exfoliated mineral graphite or a graphitized polymer film.

10. The apparatus of claim 1, wherein the resistance of the sheet of anisotropic material (70) in directions that are parallel to the front face thereof is less than 10% of the first resistance.

11. The apparatus of claim 1, wherein the at least one layer of conductive material (60) comprises hydrogel.

12. The apparatus of claim 1, wherein the at least one layer of conductive material (60) comprises a conductive adhesive.

13. The apparatus of claim 12, wherein the conductive adhesive comprises an adhesive polymer and carbon powder, particles, fibers, flakes or nanotubes.

14. The apparatus of claim 1, further comprising:
a flexible, self-adhesive backing (55) configured to support the sheet of anisotropic material (70), the at least one electrode element (E1, E2) and the at least one layer of conductive material (60) so that the front surface of the at least one layer of conductive material (60) can be positioned against the subject's skin.

15. The apparatus of claim 1, comprising a plurality of electrode elements (E1, E2).
